(19) **Europäisches Patentamt**
**European Patent Office**
**Office européen des brevets**

(11) **EP 2 243 495 A1**

(12) **EUROPEAN PATENT APPLICATION**
published in accordance with Art. 153(4) EPC

(43) Date of publication:
**27.10.2010 Bulletin 2010/43**

(51) Int Cl.:
*A61K 47/42* (2006.01)   *A61K 47/24* (2006.01)
*A61K 9/10* (2006.01)   *A61K 9/19* (2006.01)

(21) Application number: **09704056.2**

(22) Date of filing: **16.01.2009**

(86) International application number:
**PCT/CN2009/000063**

(87) International publication number:
**WO 2009/092291 (30.07.2009 Gazette 2009/31)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **16.01.2008 CN 200810010101**

(71) Applicants:
• **Shenyang Pharmaceutical University Shenyang Liaoning 110016 (CN)**
• **Hangzhou Yuhong Pharmaceutical Science & Technology Co., Ltd. Zhejiang 310012 (CN)**

(72) Inventors:
• **DENG, Yihui Liaoning 110016 (CN)**
• **DONG, Xiaohui Liaoning 110016 (CN)**
• **SHI, Li Liaoning 110016 (CN)**
• **LU, Yi Liaoning 110016 (CN)**

(74) Representative: **Hart-Davis, Jason et al Cabinet Beau de Loménie 158, rue de l'Université 75340 Paris Cedex 07 (FR)**

(54) **A DRUG DELIVERY SYSTEM, ITS PREPARATION PROCESS AND USE**

(57)   A protein-phospholipid dispersion preparation in a drug delivery system is provided, in which the weight ratio of protein to phospholipid is 1:300-300:1 and the particle size is between 5 nm and 1000 nm. The preparation process of the said preparation contains the mixture of protein phase and phospholipid phase and the homogenization process. The said drug delivery system can be used in many different pharmaceutical agents.

EP 2 243 495 A1

## Description

### Field of the invention

The present invention relates to the pharmaceutical field, and specifically relates to a drug delivery system and preparation method thereof.

### Background of the invention

In recent years, with the rapid development of drug treatment, the concept of "optimization of drug administration" has become of great concern to people; this mainly aims at reasonable reflection of the therapy value and ensuring the safety of therapeutic drugs.

Utilization of new techniques and methods to control the drug dynamic changes in the body to achieve the best therapeutic effect is performed by so-called drug delivery system (DDS). Utilization of the drug delivery system can improve the drug absorption, reduce drug toxicity and side-effects, and increase drug distribution in lesion tissue to achieve the purpose of synergism and attenuation.

In the pharmaceutics field, generally the size of nanoparticles is usually defined in the range of 1nm to 1000nm, which including submicron particles with the size over 100nm. In pharmaceutics, the nanoparticles can be classified into two categories: nano-carrier and nano-drug. Nano-carrier refers to various nano-particles in which drug is dissolved or dispersed, such as nanoliposomes, polymer nanocapsules, nanospheres, polymer micelles and so on. Nano-drug refers to the procession of nanoparticles directly from active pharmaceutical ingredients, which is essentially further development of micronization technique and micropowder technique.

Application of nanotechnology and nano-materials in pharmaceutical fields produce a nano-drug delivery system comprising nanoparticles (NP), nanospheres (NS), nanocapsules (NC), nanomicelles (NM), nano-liposomes (NL) and nano-emulsions (NE) and so on. In generally, NP are solid colloidal particles with particle size on the nanometer scale which are prepared by natural or synthetic polymer materials. NC are vesicles on the nanometer scale packed by polymer materials and have obvious vesicle shell phase. The preparation methods for NP and NC are mainly emulsion polymerization method, cross-linking polymerization method and interface polycondensation and so on. Recently it is noticeable that nano solid micelles with "core-shell" structure are formed by autopolymerization (known as self-assembly) of amphiphilic polymer materials. The hydrophilic side of micelles is towards outside and the lipophilic side is towards inside. The core can carry various drugs, enzymes and genes et al.

In the early 1970s, nanoparticles have been used as drug carriers and widely used in the pharmaceutical field for 30 years. At present, many studies have been carried out on protein nanoformulation. Proteins exhibit no immunogenicity, biodegradable and good biocompatibility effects, and protein polymers are easily metabolized and may embed drugs in relatively non-specific form, so that they can be applied for preparation of biodegradable nanoparticles. In addition, a large number of functional groups, such as amino group and carboxyl group, present in the protein molecules facilitate the drug molecules specifically binding to the surface of nanoparticles, and are suitable for the preparation of conjugates. Receptor mediated initiative target can be achieved through the surface modification to connect the appropriate ligands. Protein nanoformulation can accomplish targeted drug delivery, and also reduce the toxic and side-effects of drugs. Nanoformulations currently launched comprise, for example, paclitaxel protein nanoparticles. For example, China patent application No. CN200380109606.9, CN97199720.9, CN20031023461.X, CN02811017.X, CN03108361.7 and CN200610077006.4 and China patent No. ZL01119258.5, ZL03108361.7 all refer to protein nanoformulations. However, the products and technologies involved in these patent applications or patents only use human serum albumin (HSA) and have many disadvantages, for example used highly toxic organic solvents (e.g. chloroform, dichloromethane, etc.), long period preparation procedure and cumbersome processes. In the patent application No. CN20031023461.X, example 3 proves that addition of Tween 80 (by 1%~10%) or other surfactants such as F68\F127 et. al. may not give a good dispersion system, and drug crystals quickly crystallize from the dispersion system; example 4 proves that emulsification of drug chloroform solution only by adopting Tween 80 may not achieve uniform dispersion system, and precipitation can be found in the dispersion very soon; example 12 proves that use of water-miscible solvents only may not achieve ideal dispersion system, and the particle size distribution is very wide, where the particle size reaches a few micrometers, thereby it emphasizes specifically that use of water-miscible solvents alone should be excluded.

Liposomes are targeted drug carriers and belong to a new dosage form of fourth generation targeting drug delivery system. Liposomes were first described by British scholars, Banghatn and Standish, when phospholipid was distributed in water tested by the electron microscopy. The liposomes disperse in the water to form multilayer vesicles, and each layer is lipid bilayer; the center of vesicles and each layer are segregated by water phase, and the bilayer thickness is about 4nm. The liposomes have a membranelike structure whose inner surface is formed by a hydrophilic head of lipoid constructing the bilayer, and the lipophilic tail of the lipoid is situated in the centre of the membrane-like structure. Having the membrane-like structure, the liposomes can be filled with various types of hydrophilic, hydrophobic and amphiphilic substances which can be embedded into internal water phase of the liposomes, and inserted into the lipid bilayer or absorbed to the liposome surface. Since 1971, Englishman Rahman et al. successfully attempted to apply liposomes as drug carriers to embed amyloglucosidase for treatment of glucogenosis. As a result, researchers carried out many studies on the use of liposomes as drug carriers, and have made significant progress in preparation, stability and targeting of the liposomes.

Recently, the materials used for preparation of liposomes include natural phospholipid such as egg yolk phosphatidylcholine, soybean phosphatidylcholine, semi-synthetic phospholipids such as hydrogenated phospholipids, and synthetic phospholipids such as dipalmitoyl phosphatidylcholine, distearoyl phosphatidylcholine and so on. The main roles of liposomes comprise the following aspects: 1) liposomes are non-toxic or have low toxic and side effects on the body and may increase bioavailability, because their bilayers have greater similarity to biomembrane and histocompatibility which lead to better absorbtion by the tissues; 2) the process of liposomes embedding drugs is a physical process, which does not change the molecular structure of drugs and will not destroy the drug ingredients so that the embedded drugs may avoid being destroyed by hydrolase in vivo; 3) the embedded drugs may show lower toxicity for special regions of the body and reduce the administration dose, and achieve sustained-release and controlled-release; 4) different from viral vector, liposomes may be biodegradable in host body and have no immunogenicity; 5) in gene therapy, liposomes can easily combine gene and show highly targeting ability so that they can be applied for specific cell therapy; 6) starting marterials of liposomes are cheap so that the liposomes can be prepared on a large-scale.

Although liposomes have outstanding advantages, they still have several unsettled problems, such as 1) stability of liposomes is not high; 2) liposomes have low entrapment efficiency and drug-loading, and an ideal liposome prepared for a special drug usually needs a lot of screening; 3) the drugs loaded by liposomes are limited; 4) a large number of toxic solvents are usually used in the preparation process; 5) common liposomes mainly focus on reticuloendothelial cell-rich organs, and may not recognize caveolin in vascular endothelial.

Chinese patent application No. CN200610037609.1, CN02129204.3, CN02160028.7 and Chinese patent No. ZL94191101.2, ZL96190332.5 disclose compositions with several proteins and phospholipid. However, technical problems to be solved and technical solutions provided by these applications are essentially different from that of the present invention. These applications mainly formulate liposomes containing protein drugs or formulations containing phospholipid, that is, the protein is an effective active ingredient and its activity should as much as possible be maintained in the preparation process, and the protein is therapeutic active substance, not the system added as excipient components to be developed for reaching a specific purpose such as targeting, increasing the absorption. For example, China patent application No. CN200610037609.1 discloses that by adopting reverse phase evaporation method for removing toxic organic solvent (such as chloroform) and layer-by-layer adsorption technique, the concentration of phospholipid and protein are both between 0.1mg/ml and 1mg/ml, and the particle size is larger than 1000nm (wherein the particle size is 10micron in example 1, 20 micron in example 2, and 40 micron in example 3). The process does not adopt homogenation, and the proteins with low concentration are only simply adsorped, and they are therapeutic active substances; the purpose of China patent No. ZL96190332.5 is to apply phospholipids to protect protein drugs, and different from that of the present invention; and China patent application No. CN02129204.3 discloses surface preparation of protein and phospholipid composite membrane for drug studies by using oscillating bubble pressure method. However the method is complicated and the particle size of obtained formulations is large so that the obtained formulations may not be used as drug carriers for intravenous, oral, lung and other routes of administration. In addition, United States patent application No. US2007082838A1, filed April 12, 2007, discloses a docetaxel protein nanoformulation, and negates the value of phospholipid in example 6.

In addition, side effects appearing during administration, such as intravenous stimulation, phlebitis, pain, bone marrow suppression, neurotoxicity, allergy, inflammation, skin irritation and so on, are suspensive problems to be solved in research and development of new drug delivery systems.

**Summary of the Invention**

In summary, one purpose of the present invention is to provide a drug delivery system to effectively solve these problems. In addition, the present invention also provides a preparation method of the drug delivery system.

To achieve the above purpose, the present invention provides technical solutions described hereinafter.

In the first aspect, the present invention provides a drug delivery system comprising protein-phospholipid dispersion, wherein the weight ratio of protein to phospholipid in the protein-phospholipid dispersion is 1:300 to 300:1, and the particle size of the protein-phospholipid dispersion is between 5nm and 1000nm. In the protein-phospholipid dispersion, surface or surface and interior of phospholipids are coated by protein layer, or inner and outer of the liposome bilayer are coated by protein, or protein nanoparticles are formed in water phase of the liposomes. As mentioned above, the weight ratio of protein to phospholipid is 1:300 to 300:1, for example, can be 1:200 to 200:1, 1:100 to 100:1, 1:50 to 50:1 or 1:20 to 10:1 al. Particle size of protein-phospholipid is preferably between 20nm and 500nm. Phospholipids may be dispersed in water to form liposomes or lipid nanosphere, and may also form liposomes/lipid nanospheres (including emulsions) with other lipid components. The surface and/or interior of nanoparticles in the dispersion of the present invention are coated with protein layer by forming disulfide bonds after ultrasonic treatment, more importantly after the homogenization (such as microfluidic) treatment, to compose of stable drug carriers together with liposomes/lipid nanospheres (including emulsions), therefore the present invention provides a new drug delivery system.

Through a lot of experimental studies, the inventor surprisingly obtained a new drug delivery system by combining proteins with phospholipid (further with other lipid components) on the basis of available protein nanofomulation. Due to the unique dispersibility of phospholipid, the available technique of the protein nanofomulation become more feasible and reliable, more importantly, the addition of phospholipids (including other lipid components) greatly expanded the drug loading categories of protein nanoparticles. And with the surface of phospholipids or surface and interior of phospholipids coated by protein layer, stability of lipid nanoparticles (including emulsions)/liposomes is greatly increased. The phospholipids herein have at least two functions: dispersion and dissolution (load) of drug. Phospholipid itself is surfactant, the addition of phospholipids permits the addition of various other surfactants; and the addition of other surfactants further greatly increases the function of phospholipids, and help phospholipids loading drugs, and further reduce the particle size, and also can reduce the amount of phopholipids, thereby cut down costs and ensure that the preparation process is simple and suitable.

Different from the avaible techniques for preparing protein nanoparticles which need to have added or use toxic organic solvents, the drug delivery system of the present invention can dissolve and disperse drugs without using organic solvents. The drugs without adopting organic solvents include traditional Chinese medicine or animals and plants volatile oils or oleins, such as *chuanxiong rhizome* oil, angelica oil, zedoary turmeric oil, *brucea javanica* oil, ginger oil, *forsythia suspensa* oil, garlic oil, *radix bupleuri chinensis* oil, elemene, P-Cymene, *Eucalyptus globulus* oil, eucalyptus oil, cineole, benzoin oil, croton oil, Michelia alba flower oil, Michelia alba absolute oil, Michelia alba leaf oil, lime oil, lime oil 10×, lime oil 5×, white camphor oil, thyme oil, cedar wood oil, peppermint oil, spearmint oil, menthol, swordlike atractylodes rhizome oil, tea tree oil, petitgrain oil, tea leaves oil, neroli oil, lignaloe oil, tangerine peel oil, bitter citrus immature flower oil, bitter citrus immature leaf oil, angelica oil, ocimum oil, clove oil, clove bud oil, winter green oil, winter lemon oil, rhododendron oil, ledum oil, garlic oil, linaloe wood oil, bergamot oil, spikenard oil, patchouli oil, osmanthus fragrans flower absolute oil, cassia oil, olive oil, caraway oil, pepper oil, safflower seed oil, oleum cari, perilla oil, phellodendron chinense fruit oil, sassafras wood oil, anise oil, anethole, carrot seed oil, zanthoxylum oil, acacia farnesiana absolute oil, nut oil, mentha piperita oil, fineleaf schizonepeta oil, curcuma oil, bitter almond oil, cananga oil, labdanum absolute oil, capsicum essential oil, ginger oil, lemon oil, mandarin oil, tangerine oil, coix seed oil, spearmint oil, *forsythia suspensa* oil, *ganoderma lucidum* spore oil, aloe oil, Rome chamomile oil, sweet basil oil, tarragon oil, rose oil, wild rose absolute oil, rose crimson glory flower absolute oil, negundo chastetree oil, myrrh oil, rosemary oil, eubatus rubus rose flower absolute oil, eucalyptus citriodora oil, lemon-grass oil, lemon oil, origanum oil, sweet origanum oil, machilus leaf oil, grape seed oil, celery seed oil, Artemisia annuae oil, myristica oil, *shatian pomelo* flower absolute oil, *shatian pomelo* flower oil, litsea citrata essential oil, litsea citrata oil, Pearleverlasting, san-mou oil, common cnidium fruit oil, ginger oil, saga oil, aglaia odorate essential oil, turpentine oil, pine nuts oil, pine needle oil, pinaster seed oil, pinecone oil, cypress oil, camellia oil, sandalwood oil, sweet orange oil, sweet orange terpene, sinensal, sweet orange oil 10×, sweet orange oil 5×, chinese magnoliavine fruit oil, sweet almond oil, vetivert oil, citronella oil, mentha citrate oil, myrcia oil, clary sage oil, cardamon oil, cedar oil, fennel oil, Jasminum sambac flower absolute oil, biod magnolia flower bud oil, red cedar wood oil, lavender oil, valerian oil, elsholtzia splendens essential oil, cedar oil, tobacco flower absolute oil, coriander seed oil, coconut oil, wild rose oil, wild chrysanthemum flower absolute oil, capillary wormwood herb grass oil, pummelo pericarp oil, heartleaf houttuynia herb oil, roof iris rhizome essential oil, laurel oil, grapefruit oil, Ylang oil, oenothera biennis oil, mixed camphor wood oil, mixed lavender oil, fortune gardenia flower oil, amyris oil, purple perilla seed oil, camphor oil or thyme oil; or refined products from volatile oil, such as elemene purified from zedoary turmeric oil or other volatile oils, ligustilide purified from *Chuanxiong rhizome* oil or other volatile oils, butylphthalide purified from celery seed oil (the butylphthalide may also be obtained by synthesizing); or mixture obtained by mixing two or more volatile oils and refined products thereof in any proportion, preferred garlic oil, zedoary turmeric oil or elemene. The drugs without organic solvents further include natural or extracted, synthetic proteins/peptides drugs by biological engineering technology, such as insulin, thymopeptides, interleukin, interferon, thymopetidum or erythropoietin; the drugs without organic solvents further include drugs having properties of good solubility and interaction with phospholipid, such as silymarin (also known as silibinin) and so on.

The protein-phospholipid dispersion of the drug delivery system of the present invention is nano liquid preparation or nano solid preparation.

The protein of the drug delivery system of the present invention is selected from native proteins, synthetic proteins and derivates thereof. In a preferred embodiment, the protein is selected from, but not limited to, at least one of human albumin, bovine serum albumin, egg albumin, zein, hemoglobin and derivates thereof or related protein, albumin derivates (such as galactose glycated albumin) and modified porcine seralbumin (such as PEG modified porcine seralbumin).

The phospholipids of the drug delivery system of the present invention are selected from native, semisynthetic, total synthetic phospholipids and derivates thereof. In a preferred embodiment, the phospholipids are selected from, but not limited to, at least one of soybean phosphatidylcholine, egg yolk phosphatidylcholine, egg phosphatidyl glycerol (EPG), polyene phosphatidylcholine, phosphatidic acid, diphosphatidylglycerol, sphingomyelin, phosphatidyl serine, phosphatidylinositol, phosphatidylethanolamine, hydrogenated soybean phosphatidylcholine (HSPC), hydrogenated egg yolk phosphatidylcholine (HEPC), total synthetic phospholipids such as various synthetic C3-C30 phospholipid, for example distearoyl phosphatidylcholine (DSPC), dipalmitoyl phosphatidylcholine (DPPC), dioleoyl phosphatidylcholine (DOPC), dimyristoyl phosphatidylcholine (DMPC), dilauroyl phosphatidylcholine (DLPC), distearoyl phosphatidylglycerol (sodium salt DSPG), dipalmitoyl phosphatidylglycerol (sodium salt DPPG), L-$\alpha$-dimyristoyl phosphatidylglycerol (sodium salt DMPG), dilauroyl phosphatidylglycerol (DLPG), distearoyl phosphatidylethanolamine (DSPE), dipalmitoyl phosphatidylethanolamine (DPPE), dioleoyl phosphatidylethanolamine (DOPE), dimyristoyl phosphatidylethanolamine (DMPE), dilauroyl phosphatidylethanolamine (DLPE), bi-distearoyl phosphatidylglycerole (sodium salt), bi- dipalmitoyl phosphatidylglycerol (sodium salt), bi- dimyristoyl phosphatidylglycerol (sodium salt), bi- dilauroyl phosphatidylglycerol, distearoyl phosphatidylinositol, dipalmitoyl phosphatidylinositol, dioleoyl phosphatidylinositol, dimyristoyl phosphatidylinositol, dilauroyl phosphatidylinositol, palmitoyl oleoyl phosphatidylcholine, palmitoyl linoleoyl phosphatidylcholine, stearyl linoleoyl phosphatidylcholine, stearyl oleoyl phosphatidylcholine, arachidonic stearoyl phosphatidylcholine, 1,2-dihexanoyl-sn-glycero-3-phosphatidylcholine, 1,2-dilauroyl-sn-glycero-3-phosphatidylcholine, 1,2-dilauroyl--sn-glycero-3-phosphatidylethanolamine, 1,2-dimyristoyl-sn-glycero-3-phosphatidylcholine, 1,2-dimyristoyl-sn-glycero-3-phosphatidylethanolamine, 1,2-di-O-tetradecyl-sn-glycero-3-phosphatidylcholine, 1,2-dipentadecanoyl-sn-glycero-3-phosphatidylcholine, 1,2-dipalmitoyl-sn-glycero-3-phosphatidylcholine, 1-palmitoyl-2-oleoyl-sn-glycero-3-phosphatidylcholine, 1,2-dipalmitoyl-sn-glycero-3-phosphatidylethanolamine, 1-palmitoyl-2-linoleoyl-sn-glycero-3-phosphatidylcholine, 1,2-di-O-hexadecyl-sn-glycero-3-phosphatidylcholine, 1,2-di-O-hexadecyl-sn-glycero-3-phosphatidylethanolamine, 1,2-di-O-hexadecyl-sn-glycero-3-phosphatidylmethylethanolamine, 1,2-di-O-hexadecyl-sn-glycero-3-phosphatidyldimethylethanolamine, 1,2-diheptadecanoyl-sn-glycero-3-phosphatidylcholine, 1,2-distearoyl-sn-glycero-3-phosphatidylcholine, 1,2-dioleoyl-sn-glycero-3-phosphatidylcholine, 1,2-dilinoleoyl-sn-glycero-3-phosphatidylcholine, 1,2-dilinolenoyl-sn-glycero-3-phosphatidylcholine, 1,2-distearoyl-sn-glycero-3-phosphatidylethanolamine, 1,2-dinonadecanoyl-sn-glycero-3-phosphatidylcholine, 1,2-diarachidoyl-sn-glycero-3-phosphatidylcholine, 1,2-diarachidonoyl-sn-glycero-3-phosphatidylcholine, lysophosphatidyl choline, lysophosphatidyl ethanolamine, lysophosphatidyl inositol, lysophosphatidyl glycerol, lysophosphatidyl serine, 1,2-di-O-docosyl-sn-glycero-3-phosphatidic acid, 1-tetradecanoyl-2-hydroxy-sn-glycero-3-phosphatidylcholine, 1,2-dimyristoyl-sn-glycero-3-phosphatidic acid, 1-palmitoyl-2-hydroxy-sn-glycero-3-phosphatidylcholine, 1,2-dipalmitoyl-sn-glycero-3-phosphatidic acid, 1-palmitoyl-2-oleoyl-sn-glycero-3-phosphatidic acid, 1,2-di-O-hexadecyl-sn-glycero-3-phosphatidic acid (sodium salt), 1-stearoyl-2-hydroxy-sn-glycero-3-phosphatidylcholine, 1-oleoyl-2-hydroxy-sn-glycero-3-phosphatidylcholine, 1-linoleoyl-2-hydroxy-sn-glycero-3-phosphatidylcholine, 1,2-distearoyl-sn-glycero-3-phosphatidic acid, 1,2-dioleoyl-sn-glycero-3-phosphatidic acid, 1,2-di-O-octacosyl-sn-glycero-3-phosphatidic acid (sodium salt), 1,2-ditetradecanoyl-sn-glycero-3-phosphatidyl-rac-glycerol(sodium salt), 1,2-dipalmitoyl-sn-glycero-3-phosphatidyl-rac-glycerol(sodium salt), 1,2-distearoyl-sn-glycero-3-phosphatidyl-rac-glycerol(sodium salt), dipalmitoyl phosphatidylcholine, dipalmitoyl phosphatidylethanolamine, dipalmitoyl phosphatidylglycerole, tetramyristoyl diphosphatidylglycerol, tetralauroyl diphosphatidylglycerol, tetrapalmitoyl diphosphatidylglycerol and tetraoleoyl diphosphatidylglycerol.

Due to the limited dispersibility of phospholipid in water or oil phase and the limited ability of phospholipid dissolving drugs, the drug delivery system of the present invention may further comprise organic solvent in order to obtain the phase containing phospholipids easily or increase drug solubility. Different from previous disclosed technical solutions, the toxicity of the organic solvents adopted have been greatly reduced, which will be more favorable to reduction in drug toxicity and environment-friendly, and will also have the advantage of providing labor protection to workers in production; the organic solvents may further be water soluble solvents. The organic solvents used in the preferred embodiment of the present invention may be selected from at least one of ethanol, methanol, propanol, butanol (including n-butanol, *tert-butyl* alcohol), acetone, methyl-2-pyrrolidone (such as N-methyl-2-pyrrolidone or 1-methyl-2-pyrrolidone), ethyl acetate and isopropyl ether; most preferably, the organic solvent is ethanol or *tert-butyl* alcohol.

In order to expand the varieties of dissolving drugs in phospholipid, the drug delivery system of the present invention may also comprise appropriate oils (olein) or other lipids, the weight ratio of which to phospholipid is from 1: 0.1 to 1:10. The present invention develops a study on the effect of various oil phases on granularity and freeze-thaw stability of nanoparticle prepared from various amounts of MCT. In a preferred embodiment, the oils or other lipids are selected from at least one of medium chain triglyceride (MCT), structured-oil (structured-triglyceride), tocopherol, tocopheryl acetate, oleinic acid, ethyl oleate, soybean oil, safflower oil, olive oil, octanoic acid and esters thereof, decanoic acid and esters thereof, lauric acid and esters thereof, palmitinic acid and esters thereof, linoleic acid and esters thereof, linolenic acid and esters thereof, docosahexaenoic acid and esters thereof, deep-sea fish oil and plant volatile oil. In a more preferred embodiment, the oil substance is selected from MCT, oleinic acid and esters thereof or structured-oil (structured-triglyceride).

In order to improve the stability of the drug delivery system, it may further comprise antioxidants which include water-soluble antioxidant and oil-soluble antioxidant, and further include inert gas. In a preferred embodiment, water-soluble antioxidants are selected from at least one of Vitamin C and esters thereof, sodium sulfite, sodium bisulfite, sodium metabisulphite, sodium hyposulfite, thioctic acid, cystine, cysteine, histidine and glycine. In a preferred embodiment, oil-soluble antioxidant is selected from BHA, BHT, tocopherol or thioctic acid and so on.

In a preferred embodiment of the present invention, oil is MCT, the weight ratio of which to phospholipid is 1:1; in a further preferred embodiment, oil is structured-oil, the weight ratio of which to phospholipid is 1:1.7.

The drug delivery system of the present invention may further comprise cholesterol and derivates thereof (such as cholesteryl hemisuccinate) and/or other lipids such as sitosterol. When preparing phospholipid phase, cholesterol and/or sitosterol is added as additive of liposomes or emulsions, and these lipids may be used to assist phospholipid to load lipophilic substance.

In a preferred embodiment of the present invention, it may further comprise cholesterol.

The drug delivery system of the present invention may further comprise ligand and/or antibody, wherein the ligand is selected from galactose derivates, transferrin derivates, folic acid and derivatives thereof, glucosamine derivates and various RGD and derivatives thereof and so on. The antibody is selected from various murine, humanized, recombine murine or recombinant humanized antibodies. The preferred ligand is selected from galactose ligand, transferrin, folic acid and RGD (tripeptide structure of Arg-Gly-Asp) and derivatives thereof. Since the drug delivery system of the present invention comprises phospholipid or other lipids, the lipid substance may be used to load some ligands, antibodies containing lipophilic groups and derivatives thereof to achieve the purpose of active targeted drug delivery.

In a preferred embodiment of the present invention, it may further comprise cholesterol derivatives of galactose disclosed in Chinese patent application No. CN200610121794.2 filed by the present inventor.

In order to achieve specific target or cell fusion, improvement efficiency of gene transfection, the drug delivery system of the present invention also comprises positively charged substances (cation), such as fatty amine, polyamine, TC-Chol (polyamine cationic lipids), DC-Chol, cationic cardiolipin, chitosan or octadecylamine (hydrogenated tallow amine kiber) and so on.

In order to improve drug or compound stability and ensure the formulation in a reasonable range of pH values, the drug delivery system of the present invention may further comprise pH regulator, wherein the pH regulator is selected from at least one of organic acid, organic base, inorganic acid or inorganic base, such as citric acid, lactic acid, fumaric acid, tartaric acid, acetic acid, glucanic acid, lactobionic acid, sorbic acid, succinic acid, maleic acid, ascorbic acid, oxalic acid, formic acid, benzenesulfonic acid, benzoic acid, aspartic acid, hydrochloric acid, sulphuric acid, phosphoric acid, nitric acid, sodium hydroxide, arginine, lysine and so on.

A preferred embodiment of the present invention may further comprise citric acid.

In the second aspect, the present invention provides a method for manufacturing the above drug delivery system comprising the following steps: 1) preparing phospholipid-containing phase (the phospholipid phase is obtained by the methods such as directly use of phospholipid, mixing phospholipid with oil et. al to dissolve, or solubilization of phospholipid and other ingredients in organic solvent); 2) preparing protein-containing aqueous phase; 3) mixing phospholipid-containing phase and protein-containing aqueous phase, and extruding optionally at 0-60°C under a pressure of 400-40000psi (pound/square inch), then homogenizing. Where the extrusion step is necessary, one can carry out extrusion by using extruder to obtain liposome to afford desirable paricle size and reduce cycle times. Extrusion is particularly suitable for loading heat-sensitive and pressure sensitive active substance. Homogenization will be carried out after extrusion.

Different from the prior art, phospholipid-containing phase of the present invention may be prepared by mixing the drug, phospholipid and oil directly to dissolve without organic solvent.

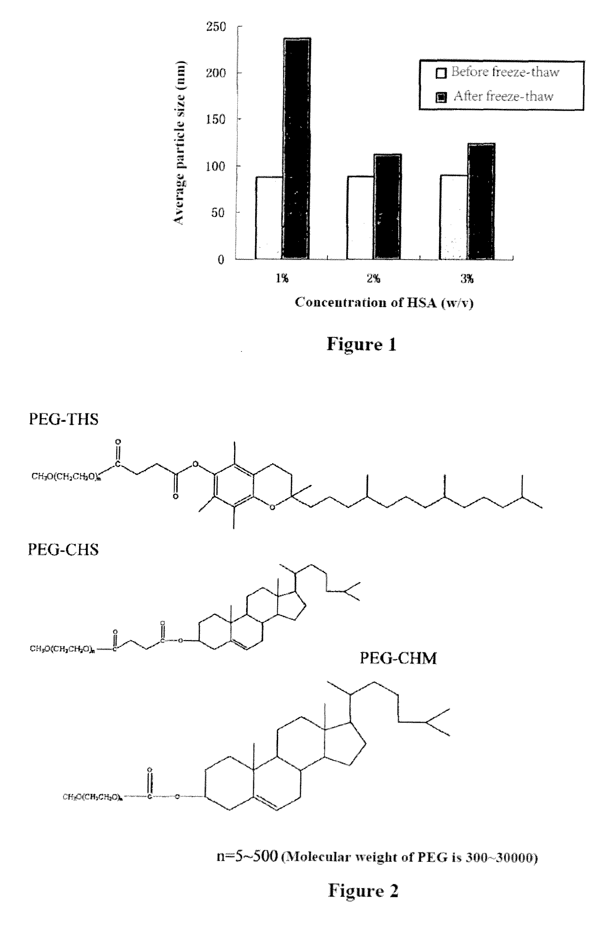

The protein-containing aqueous phase disclosed in the preparation method of the present invention is 0.005-50% (w/v) protein aqueous solution, preferred 0.05-30%(w/v), more preferred 0.1-10%(w/v). The effect of 1-5%HSA (human serum albumin)-containing aqueous phase on the particle diameter and freeze-thaw stability of nanoparticle prepared from various concentrations of HAS have been further illustrated in the present invention. Please see figure 1 and the corresponding embodiment. The protein of the preparation method of the present invention is selected from native protein, or synthetic protein and derivates thereof. In a preferred embodiment, the protein is selected from at least one of human albumin, bovine serum albumin, ovalbumin, hemoglobin and derivates thereof, albumin derivates (such as albumin glycated with galactose) and modified porcine albumin (such as PEG-modified porcine albumin).

Phospholipid disclosed in the preparation of phospholipid-containing phase of the present invention is selected from natural, semisynthetic, total synthetic phospholipid and derivates thereof. In a preferred embodiment, the phospholipid is selected from, but not limited to, at least one of the following: soybean phosphatidylcholine, egg yolk phosphatidylcholine, egg phosphatidyl glycerol (EPG), phosphatidic acid, diphosphatidylglycerol, sphingomyelin, phosphatidyl serine, phosphatidylinositol, phosphatidylethanolamine, hydrogenated soybean phosphatidylcholine, hydrogenated egg yolk phosphatidylcholine, distearoyl phosphatidylcholine, dipalmitoyl phosphatidylcholine, dioleoyl phosphatidylcholine, dimyristoyl phosphatidyl choline, dilauroyl phosphatidylcholine, distearoyl phosphatidylglycerol and sodium salt thereof, dipalmitoyl phosphatidylglycerol and sodium salt thereof, L-α-dimyristoyl phosphatidylglycerol and sodium salt thereof, dilauroyl phosphatidylglycerol, distearoyl phosphatidylethanolamine, dipalmitoyl phosphatidylethanolamine, dioleoyl phosphatidylethanolamine, dimyristoyl phosphatidylethanolamine, dilauroyl phosphatidylethanolamine, bi- distearoyl phosphatidylglycerole and sodium salt thereof, bi- dipalmitoyl phosphatidylglycerol and sodium salt thereof, bi-dimyristoyl phosphatidylglycerol and sodium salt thereof, bi- dilauroyl phosphatidylglycerol, distearoyl phosphatidylinositol, dipalmitoyl phosphatidylinositol, dioleoyl phosphatidylinositol, dimyristoyl phosphatidylinositol, dilauroyl phosphatidylinositol, palmitoyl oleoyl phosphatidylcholine, palmitoyl linoleoyl phosphatidylcholine, stearyl linoleoyl phosphatidylcholine, stearyl oleoyl phosphatidylcholine, arachidonic stearoyl phosphatidylcholine, DSPE-PEG, DPPE-PEG, DMPE-PEG and DLPE-PEG, wherein the molecular weight of PEG in DSPE-PEG, DPPE-PEG, DMPE-PEG and DLPE-PEG is from 100 to 10000. The phospholipid may also be selected from other PEG derivates such as PEG-THS, PEG -CHS, PEG-CHM, whose structure is shown in figure 2.

In a preferred embodiment, the homogenization disclosed in the present invention is high-pressure homogenization or microfluidic homogenization, wherein the operating steps of high-pressure homogenization include steps wherein the crude emulsion is high-pressure homogenized under a pressure of 1,000-40,000 psi and the high-pressure homogenization is repeated for 1-20 cycles to form the desired emulsion. The pressure is preferably 2,000-20,000 psi, and more preferably 8,000-16,000 psi. The crude emulsion is preferably repeated 1-15 cycles, and more preferably 1-5 cycles.

In embodiments, the inventor points out the reasons of preparing nanoparticle formulation without using ultrasonic probe, and at the same time demonstrates the significance of phospholipid which is completely different from the effect of phospholipid in United States patent application No. US2007082838A1. The inventor also demonstrates that addition of other surfactants or lipid substance to the phospholipid is reasonable, which is completely different from the effect of phospholipid in Chinese patent application No. CN20031023461..X.

High-pressure homogenization or microfluidic homogenization is applied in a preferred embodiment of the present invention; alternatively homogenization is applied after extrusion which is to control any desired paticle size.

The preparation method of the present invention may also comprises freeze drying or spray drying, and the obtained solid or powder may be further prepared into other desired preparations, wherein the excipient used in the freeze drying or spray drying method is selected from at least one of sucrose, lactose, glucose, mannitol, dextran, trehalose, xylitol, maltose, fructose, protein, chitosan, glycine, citral and sodium chloride.

It is more important to use suitable cryoprotectants (freeze-dry excipient) during freeze drying. The cryoprotectants may not only play as a skeleton frame of freeze-dry preparations, but also promote the microparitcle preparation to disperse again.

Freeze-drying is divided into two stages: sublimation drying (first stage of drying) and desorption drying (second stage of drying). Duration of sublimation drying relates to product varieties and thickness of subpackage. About 95% of all water is removed in first stage of drying. It may be observed that sublimation interface gradually moves to the bottom of the container and disappears after the first stage of drying is completed. The second stage of drying is mainly to remove part of crystallization water and hydration water absorbed in lattice gap of a solid substance or hydration water bound to some polar groups with hydrogen bonds. This part of hydration water having high adsorption energy may be desorbed by offering sufficient energy (adequate temperature and vacuum).

It is found in experiments that if the drying duration of the first stage is too short, then ice crystals do not completely sublimate and affect the appearance of freeze-dried products. The freeze-dried products with smooth appearance and porous texture may be obtained when the duration of sublimation drying is 12 hours and then desorption drying is for 2-3 hours. The obtained freeze-dried products may quickly disperse after the water addition.

From the above studies, the determined freeze-drying process finally is as follows: first, pre-freezing samples at -74°C for 8h; and vacuumizing at -35°C for 20 minutes to reach a vacuum degree of 15 Pa; and raising the temperature to -20°C and then maintaining 12h (in this stage, most of water in the samples will be removed); and finally raising the temperature to 10°C and maintaining 2-3h (the residual water in the samples will be removed in this stage).

Studies have been performed on use and combined use of cryoprotectant in preferred embodimens. See content of the corresponding embodiment for concrete results.

A preferred embodiment of the present invention may further comprise freeze drying which selects sucrose, trehalose, lactose, xylitol and maltose etc. as freeze-drying excipient.

The preparation method of the present invention may further comprise filtration sterilization. The obtained nanoparticles and emulsions of the present invention are very easy to filter sterilize by passing through a porous membrane. However, when docetaxel albumin nanoparticle is prepared which is disclosed in United States patent application US2007082838A1, the nanoparticle having nonuniform granularity (and the inventor has repeatedly proved that the method described in this patent application has this problem) needs to be filtered respectively through a microporous membrane with pore size ratings in the range of 1μm, 0.8μm, 0.6μm, 0.4μm and 0.2μm to remove macroparticle gradually and accomplish ultimately the purpose of 0.2μm filtration sterilization. The process of the United States patent application not only shows disadvantages of complicated operation process and is time-consuming, but also may lead to a series of problems such as loss of drug content, fluctuations between different production batches, and is difficult to reproduce etc.

A preferred embodiment of the present invention may further comprise using $0.2\mu$m microporous membrane for filtration sterilization.

The drug delivery system of the present invention may be used to reduce at least one side effect of a drug. The side effect includes intravenous stimulation, phlebitis, pain, bone marrow suppression, neurotoxicity, allergy, inflammation, skin irritation etc.

The drug delivery system of the present invention may be administrated via several drug administration routes, such as injection (comprising intravenous, arterial, intra-articular cavity, subcutaneous, intradermal, intramscular etc.), oral (comprising sublingually, oral mucosa), intra-cavity (including ophthalmic suppositories, nasal suppositories, audi suppositories, tracheal suppositories, lung suppositories, rectal suppositories, vaginal suppositories, urethral suppositories etc.) and cutaneous etc. The preparations suitable for vaginal administration may be vaginal suppository, cotton wool tampon, creams, gelata, pastes, foam or spray, and comprising active substance and a suitable carrier known in the art.

The drug (generalized) can be designed in the drug delivery system of the present invention comprising pharmacologically active substances and pharmaceutical acceptably excipient thereof. The pharmacologically active substances comprise drugs (defined narrowly), diagnostic reagent and nutrient substance, wherein the drugs are selected from anti-cancer drugs, narcotic drugs, cardiovascular drugs, antihypertensive drugs, antiinflammatory agent, antiarthritic, antiasthmatic drugs, analgesic, immunosuppressive agents, antifungal agent, anti-arrhythmia drugs, antibiotics, steroids etc.

The designed drugs in the drug delivery system are selected from, but not limited to, the following materials: analgesic agent/febrifuges (such as aspirin, paracetamol, ibuprofen, naproxen sodium, buprenorphine hydrochloride, darvon, propoxyphene napsylate, pethidine hydrochloride, dihydro-morphinone hydrochloride, morphine sulfate, oxycodone hydrochloride, codeine phosphate, dihydrocodeine bitartrate, pentazocine hydrochloride, dihydrocodeinone bitartrate, levorphanol tartrate, diflunisal, trolamine salicylate, nalbuphine hydrochloride, mefenamic acid, butorphanol tartrate, choline salicylate, butalbital, phenyltoloxamine citrate, diphenhydramine citrate, levomeprazin, cinnamyl ephedrine hydrochloride, xalogen etc.); anesthetic (such as cyclopropane, enflurane, fluothane, isoflurane, methoxyflurane, nitric oxide, propofol (disoprofol) etc.); antiasthmatic agents (such as azelastine, ketotifen, Traxanox etc.); antibiotics (such as neomycin, streptomycin, chloramphenicol, cephalosporin, ampiopen, penicillin, tetracycline etc.); antidepressant (such as nefopam, oxypertine, doxepin hydrochloride, amoxapine, trazodone hydrochloride, amitriptyline hydrochloride, maprotiline hydrochloride, phenelzine sulfate, desmethvlimipramine hydrochloride, nortriptyline hydrochloride, tranylcypromine sulfate, fluoxetine hydrochloride, doxepin hydrochloride, imipramine hydrochloride, imipramine salicyl salicylate, nortriptyline, amitriptyline hydrochloride, isocarboxazid, desmethvlimipramine hydrochloride, trimipramine maleate, protriptyline hydrochloride etc.); antidiabetic agents (such as biguanides, hormone, sulfonylurea derivates etc.); antifungal agents (such as griseofulvin, ketoconazole, amphotericin B, nystatin, candicidin etc.); antihypertensive agents (such as propranolol, propafenone, oxprenolol, nifedipine, reserpine, trimethaphan camphorsulfonate, phenoxybenzamine hydrochloride, pargyline hydrochloride, deserpidine, diazoxide, guanethidine sulfate, clonidine, rauwolfine, sodium nitroprusside, ajimalinin, alseroxylon, phentolamine mesylate, reserpine etc.); antiinflammatory agent (such as (non-steroidal) indomethacin, naproxen, ibuprofen, ramifenazone, piroxicam; (steroids) corticosterone, dexamethasone, dexamethasone palmitate, flumethasone, hydrocortisone, prednisolone, prednisone etc.); antineoplastic agents (such as anthracycline antibiotics, doxorubicin hydrochloride, cyclophosphamide, actinomycin, bleomycin, rubomycin, adriamycin, epirubicin, mitomycin, methotrexate, 5-fluorouracil, platinum drugs such as carboplatin, oxaliplatin, eptaplatin, platinum acetate, cisplatin, bischloro-nitrosourea (BCNU), methyl-CCNU, etoposide, etoposide, interferon, camptothecin and derivates thereof, phenesterin, taxol and derivates thereof, docetaxel and derivates thereof, vinblastine, vincristine, vinorelbine and derivates thereof, tamoxifen, piposulfan, tetracyclic triterpenoids etc.); antianxiety agents (such as lorazepam, buspirone hydrochloride, prazepam, chlordiazepoxide hydrochloride, oxazepam, dipotassium clorazepate, diazepam, hydroxyzine pamoate, hydroxyzine hydrochloride, alprazolam, droperidol, halazepam, chlormethazanone, dantrolene etc.); immunosuppressive agents (such as cyclosporine, azathioprine, mizoribine, FK506(tacrolimus) etc.); antimigraine agents (such as erogtamine tartrate, propranolol chloride, isometheptene mucate, bonadorm etc.); sedative/hypnotic (such as barbiturates (such as pentobarbital, pentobarbital sodium, secobarbital sodium); benzodiazepines (such as flurazepam hydrochloride, triazolam, tomazeparm, midazolam hydrochloride et al.)); anti-anginal drug (such as β-adrenergic antagonists; calcium channel blockers (such as nifedipine, deltiazem hydrochloride etc.); nitrates (such as nitroglycerin, isosorbide dinitrate, pentaerithrityl tetranitrate, erythrityl tetranitrate etc.)); antipsychotic drug (such as haloperidol, loxapine succinate, loxapine hydrochloride, thioridazine, mellaril, tiotixene, fluphenazine hydrochloride, fluphenazin decanoate, fluphenazin enanthate, trifluoperazine hydrochloride, chlorpromazine hydrochloride, perphenazine, lithium citrate, prochlorperazine etc.); antimanic agents (such as lithium carbonate); anti-arrhythmia agents (such as bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide, tocainide hydrochloride, lidocaine hydrochloride etc.); anti-arthritic (such as phenylbutazone, sulindac, penicillamine, salsalate, piroxicam, azathioprine, indomethacin, meclofenamate sodium, gold sodium thiomalate, ketoprofen, auranofin, aurothioglucose, tolmetin etc.); gout suppressants (such as colchicine, allopurinol etc.); anticoagulant agent (such as heparin, heparin sodium, warfarin sodium etc.); thrombolytic agents (such as urokinase, streptokinase, recombinant plasminogen activators etc.), argatroban; antifibrinolytics (such as aminocaproic acid); hemorheologic agent (such as pentoxifylline); antiplatelet drug (such as aspirin, empirin, ascriptin etc.); anticonvulsant (such as valproic acid, sodium valproate, phenytoin, phenytoin sodium, clonazepam, primidone, phenobarbital, phenobarbital sodium, carbamazepine, amobarbital sodium, mesuximide, mesuximide, methylphenobarbital, mephenytoin, phensuximide, paramethadione, ethotoin, phenacemide, secobarbital sodium, clorazepate dipotassium, trimethadione etc.); anti-parkinson drugs (such as acetamide etc.); antihistaminics/antipruritics (such as hydroxyzine hydrochloride, diphenhydramine hydrochloride, chlorphenamine, brompheniramine maleate, cyproheptadine hydrochloride, terfenadine, tavehil, triprolidine, carbinoxamine maleate, diphenylpyraline hydrochloride, phenindamine, azatadine maleate, pyribenzamine, dexchlorpheniramine maleate, methdilazine hydrochloride, trimeprazine tartrate etc.); calcium regulators (such as calcovarin, parathyroid hormone etc.); anti-bacterial agent (such as amikacin sulfate, aztreonam, chloramphenicol, chloramphenicol palmitate , chloramphenicol sodium succinate, ciprofloxacin hydrochloride, clindamycin hydrochloride, clindamycin palmitate, clindamycin phosphate, metronidazole, metronidazole hydrochloride, gentamycin sulfate, lincomycin hydrochloride, tobramycin sulfate, vancomycin hydrochloride, colistin sulfate B, colistimethate sodium, colistin sulfate E etc.); antiviral drugs (such as γ-interferon, azidothymidine, amantadine hydrochloride, ribavirin, acycloguanosine, entecavir etc.); antimicrobial agent (such as cephalosporin (such as cefazolin sodium, cefradine, cefaclor, cefapirin sodium, ceftizoxime sodium, cefoperazone sodium, cefotetan disodium, cefuroxime, cefotaxime sodium, cefadroxil monohydrate, ceftazidime, cefalexin, cefalotin sodium, cefalexin hydrochloride monohydrate, cefamandole nafate, cefoxitin sodium, cefonicid monosodium, ceforanide, ceftriaxone sodium, ceftazidime, cefadroxil, cefradine, cefuroxime sodium etc.); penicillins (such as ampicillin, amoxicillin, benzathine benzylpenicillin G, chloroxacillin, ampicillin sodium, penicillin G potassium, penicillin V potassium, piperacillin sodium, oxacillin sodium, bacampicillin hydrochloride, cloxacillin sodium, ticarcillin sodium, azlocillin sodium, carbenicillin indanyl sodium (carindacillin), penicillin G potassium, procaine benzylpenicillin G, methicillin sodium, nafcillin sodium etc.); erythromycins (such as erythromycin ethylsuccinate, erythromycin, erythromycin estolate, erythrocin lactobionate, erythromycin stearate, erythromycin ethylsuccinate etc.); tetracyclines (such as tetracycline hydrochloride, doxycycline hyclate, minocycline hydrochloride etc.), etc.); anti-infective agents (such as GM-CSF); bronchial-dilating agents (such as sympathomimetic agents (such as epinephrine hydrochloride, metaproterenol sultate, terbutaline sulphate, isoetharine, isoetharine mesylate, isoetharine hydrochloride, salbutamol sulfate, salbutamol, bitolterol mesylate, isoprenaline hydrochloride, terbutaline sulphate, epinephrine bitartrate, metaproterenol sultate, epinephrine, epinephrine bitartrate); anticholinergic agents (such as ipratropium bromide); xanthines (such as aminophylline, diprophylline, metaproterenol sultate, aminophylline); mast cell stabilizers (such as sodium cromoglicate); inhaled corticosteroids (such as flunisolide, beclometasone, beclometasone dipropionate monohydrate), salbutamol, beclometasone dipropionate (BDP), ipratropium bromide, salbutamol sulfate aerosol, ketotifen, salmeterol, xinafoate, terbutaline sulphate, triamcinolone, aminophylline, nedocromil sodium, metaproterenol sultate, salbutamol sulfate, flunisolide etc.); hormone (such as androgens (such as danazol, testosterone cypionate, fluoxymesterone, ethyltestosterone, testosterone heptanoate, methyltestosterone, fluoxymesterone, testosterone cypionate), estrogens (such as estradiol, estrone, conjugated estrogens), progestogens (such as methoxyprogesterone acetate, norethisterene acetate), corticosteroids (such as triamcinolone, betamethasone, betamethasone sodium phosphate, dexamethasone, dexamethasone sodium phosphate, dexamethasone acetate, prednisone, methylprednisolone suspensions, triamcinolone acetonide, methylprednisolone, methylprednisolone sodium phosphate, methylprednisolone sodium succinate, hydrocortisone sodium succinate, methylprednisolone sodium succinate, aristocortacetonide, hydrocortisone, hydrocortisone cypionate, prednisolone, fluorohydrocortisone acetate, paramethasone acetate, prednisolone butylacetate, prednisolone acetate, prednisolone sodium phosphate, hydrocortisone sodium succinate etc.), thyroid hormones (such as levothyroxine sodium et al.) etc.); hypoglycemic agents (such as human insulin, purified bovine insulin, purified porcine insulin, glibenclamide, chlorpropamide, glipizide, tolbutamide, tolazamide etc.); reduce blood cholesterol (such as atromide, dextrothyroxine sodium, probucol, lovastatin, nicotinic acid etc.); proteins (such as desoxyribonuclease, alginase, superoxide dismutase, lipase etc.); nucleic acid (such as positive-sense or antisensenucleic acids encoding any of therapeutic proteins including any proteins mentioned herein etc.); erythropoiesis-stimulating drugs (such as erythropoietin); antiulcer/ anti-reflux drugs (such as famotidine, cimetidine, ranitidine hydrochloride etc.); anti-nausea/antiemetic drugs (such as meclozine hydrochloride, nabilone, prochlorperazine, dimenhydrinate, promethazine hydrochloride, thiethylperazine, scopolamine et al.); fat-soluble vitamins (such as vitamin A, D, E, K etc.); calcitriol; as well as other drugs such as mitotane, nitrosourea, elliptinium acetate et al. The said designed drugs may further be selected from verteporfin, bufogenin, bufalin, triptolide, cidofovir, bicyclol and derivates thereof, bifendate and derivates thereof, glycyrrhizic acid, oxymatrine, amlodipine, levothyroxine sodium, dihydroergotoxine mesylate, butoconazole nitrate, meloxicam, rubitecan, pemetrexed, monostalotetrahexosylgangliside GM1, puerarin, gemcitabine, Huperzine A, Bulleyaconitine A, lidamycin, sea cucumber extract, dried Chinese forest frog etc.

HSA has been disclosed to be used as emulsifier for emulsifying oil phase such as chloroform so that protein nanoparticles are formed through disulfide bond cross-link among HSA proteins. A problem of the prior art is that it is difficult to obtain stable nanoparticles with minor granularity.

In a preferred embodiment of the present invention, drugs are respectively selected from drugs such as cucurbitacin, docetaxel, 10-hydroxycamptothecin, amphotericin B, propofol, entecavir and paclitaxel et al.

In addition, drugs may be loaded by using techniques of ammonium sulfate gradient method, pH gradient method, ion gradient method for liposomes structure in the protein-phospholipid preparation. The preferred drugs may be selected from adriamycin, epirubicin, topotecan, mitoxantrone, vincristine or vinorelbine etc.

The drug delivery system of the present system may be applied to human or animals via any appropriate administration routes. A preferred embodiment is that pharmaceutical compositions containing the drug delivery system of the present system may be administrated via an intravenous, intra-arterial, oral, intratracheal, subcutaneous, intraocular, intravesical or cutaneous route.

Despite our best efforts, we can not obtain the protein nanoparticles disclosed in Chinese patent application No. CN20031023461.X by using problematic methods disclosed in the said document. Moreover, prior processes use a large number of toxic organic solvents, for example chloroform, dichloromethane. Allowing for environment protection and labor insurance for staff, the inventor establishes a technology platform which may greatly reduce the use of poisonous solvents or even without using poisonous solvents. In addition, application of phospholipids in the invention allows enlarging greatly drug-loading scope of the protein nanoparticles and reducing granularity thereof, thus filtration sterilization may be performed with microporous membrane. Moreover, addition of phospholipids or other lipids in the formulation may allow loading ligands, antibodies and derivates thereof having lipophilic groups by utilizing these lipid components to accomplish active targeted drug delivery. Through a large number of long-term trials, we found that binding of phospholipid and protein may obtain the surprising result that the stability of the prepared nanoformulations has been greatly improved and their granularity can be freely adjusted. More importantly, it can insert active identification head group of various ligands, antibodies and so on into nanoformulation to obtain a more useful and meaningful drug delivery system.

Especially for tumors, HAS can bind with albumin receptors Gp60 in endothelial cell membrane and activate caveolae-mediated albumin transport to carry drugs through the vascular endothelium. SPRAC (Secreted Protein Acidic and Rich in Cystein) expression is usually enhanced in tumor tissue. SPRAC is a kind of non-structural cytoskeletal protein, and is involved in interaction of cell matrix during tissue shape and embryo development and cancer invasion and metastasis. SPRAC and Gp60 have homologous sequences and may also combine with albumin, thus result in easy accumulation of HSA nanoparticles in tumor tissue to form drug pool and improve the drug targeting anti-tumor cells. Cell culture tests in vitro also show that the HSA nanoparticles increased the affinity of drug to tumor cells.

In addition, with the help of phospholipid, the drug delivery system of the present invention can be used alone with aqueous phase dispersion without adding solvent to easily achieve nanodispersion.

A preferred embodiment of the present invention respectively selects human serum albumin (HSA), bovine serum albumin, porcine haemproteins, egg albumin, zein and PEG porcine haemproteins and so on.

In a preferred embodiment of the present invention, egg yolk phosphatidylcholine (EPC) is selected; in another preferred embodiment, soybean phosphatidylcholine (SPC) is selected; in another preferred embodiment, dimyristoyl phosphatidyl choline (DMPC) and L-$\alpha$-dimyristoyl phosphatidylglycerol (DMPG) are selected; in another preferred embodiment, HSPC and DSPG are selected; in another preferred embodiment, lecithin and DOPC are selected; in another preferred embodiment, lecithin is selected; in another preferred embodiment, L-$\alpha$-dimyristoyl phosphatidylglycerol (DMPG) is selected; in another embodiment, TPGS, PEG-CHS and so on are selected.

In a preferred embodiment, ethanol is selected; in another preferred embodiment, methyl-2-pyrrolidone or methyl-2-pyrrolidone/methanol mixed solvent is selected; in another embodiment, methanol is selected; in another embodiment, ter-butyl alcohol is selected; in another embodiment, isopropyl ether is selected.

Comparing with the prior arts, the present invention has the advantages of:

1) combination of phospholipid and protein has greatly enhanced the feasibility of industrialization of preparation, particularly using organic solvents with low toxicity without using poisonous or toxic organic solvents, even without using organic solvents;

2) combining with liposome technology, the surface or the surface and internal surface of liposome are coated/covered by protein layers cross-linked with disulfide bond, and thus greatly enhancing the stability of liposomes;

3) utilization of characteristics of liposome has greatly enlarged the drug-loading scope of the protein nanoparticles, for example loading vincristine sulphate, adriamycin, topotecan, thymopetidum, insulin, bFGF and so on (the prior patents, such as CN03108361.7A, US2007082838A1, are only suitable for water-insoluble drugs);

4) when preparing the nanoparticle-containing proteins by using the prior technique, the dispersion which is cycled for many times, such as for at least 5 times under high pressure (such as the prior technique disclosed in Chinese patent application No.CN200310123461.X), or for more than 15 times, will increase the hazard of protein denaturation and cost. However, the techniques of the present invention can reduce the cycles, and the optimal formulation process only needs to be cycled for 1 to 5 times;

5) as for the dispersion pressure, the process of prior art needs pressure greater than 20000psi, the optimal process of the present invention only needs pressure less than 20000psi to obtain a disperse system with the same or smaller particle size;

6) the liquid-type drug delivery system containing phospholipid-protein can be prepared through utilization of the good dispersion ability of phospholipid. Compared with the prior art which is only stable for a few hours, the prepared drug delivery system has better stability which may last for more than tens of hours, or even for tens of days.

7) Phospholipid themselves are surfactants. The addition of phospholipid allows adding of other surfactants into the whole system, and further significantly reduces the particle size, amount of phospholipid, cost and also ensure that the preparation method of drug delivery systems is easy and applicable.

8) as reasonably using phospholipid, it can insert various ligands and derivates thereof, antibodies and derivates thereof into nanoparticles by optionally using extra-insertion method to further improve targeting.

9) as reasonably using phospholipid, it can prepare nanoparticles containing oils first or combining with extrusion technology, i.e. firstly particle size is reduced under high pressure or by using extrusion technology to obtain optionally the desired particle size, and secondly the proteins are extrapolated. The prepared protein nanoparticles are dispersed only for 1 to 2 cycles under high pressure which may significantly reduce protein denaturation and ensure activity of proteins, and also greatly reduce amount of proteins.

10) as reasonably using phospholipid, phospholipid, proteins, excipients and drugs may be mixed and dispersed, especially be extruded by using extrusion technology to be reduced to the desired particle size under mild conditions, and finally be dispersed under low pressure to prepare the desired protein nanoparicle (protein nano-vesicles) (for example, phospholipid is hydrated by using water phase containing proteins, then the mixture is extruded by controlling any of the desired granularity, and then be homogenized). The preparation method of the present invention is suitable for the pressure-sensitive and/or heat-sensitive drugs.

**Description of the Drawings**

Figure 1 shows the study results of freeze-thaw experiment on granularity of CuB (cucurbitacin B) preparation containing various concentration of HSA.

Figure 2 shows the structural formula of PEG-THS, PEG-CHS and PEG-CHM.

Figure 3 shows the particle size distribution of nano-preparation in example 12 of the present invention.

Figure 4 shows the particle size distribution of nano-preparation standed for 30 days in example of the present invention.

Figure 5 is a photograph of the nanoparticle size in example 25 of the present invention.

Figure 6 is a photograph of the liposomes in example 32 of the present invention.

Figure 7 is a schematic diagram of the effect of cucurbitacin B solution of the present invention on proliferation of Hep-2 cell (human laryngocarcinoma epithelial cells).

Figure 8 is a schematic diagram of the effect of CuB HSA nanoparticles and solution of CuB /HSA of the present invention on proliferation of Hep-2 cell (human laryngocarcinoma epithelial cells) at various dosages.

Figure 9 is a schematic diagram of the effect of CuB HSA nanoparticles and solution of CuB/HSA of the present invention on proliferation of A549 cell (human lung adenocarcinoma cell) at various dosages.

Figure 10 is a schematic diagram of the effect of CuB HSA nanoparticles and solution of CuB/HSA of the present invention on proliferation of HepG2 cell (human hepatoma carcinoma cell) at various dosages.

Figure 11 is a schematic diagram, compared with emulsion, liposomes, of inhibition of different tumor cells growth by cucurbitacin B HSA nanoparticles.

**Detailed description of the Invention**

The invention will be described in details with reference to the following examples. It should be understood, the following examples are used for illustrating the invention but not intended to limit the invention to these examples. The present invention encompasses various modifications and/or alterations consistent with the spirit of the present invention that will be appreciated by those skilled in the art.

Example 1: Issues in the preparation of cucurbitacin HSA nanoparticle by ultrasound probe sonography

| Cucurbitacin B | 0.01g |
| HSA | 4g |
| Water | appropriate amount |

Cucurbitacin B (purity >98%) was dissolved in 1ml ethanol for standby. An HSA solution of 4% (g/ml) was obtained by dissolving the formulation amount of HSA in water and added into the ethanol solution of cucurbitacin B. After being dispersed, the obtained mixed solution was placed in an ultrasonic machine and treated by 200W ultrasound, followed by dispersion for 5 minutes by 600W ultrasound to obtain an emulsion. The organic solvent was removed by rotary evaporation, and the nanoparticles were obtained. The average particle size of the prepared nanoparticles was 282.2nm, with the particle distribution too wide to pass through $0.4\mu m$ micropore film. After being left to stand for 20 minutes, precipitate was produced, thereby indicating that the nanoparticle system was instable. After freeze-drying, the system was poorly redispersible, and hard to return to the state before freeze-drying. The average particle size after redispersion was greater than 400nm.

Example 2: Resolution of issues in the preparation of cucurbitacin HSA nanoparticle with ultrasound probe sonography by addition of phospholipid

| Cucurbitacin B | 0.01g |
| HSA | 4g |
| S100 | 1.5g |
| Water | appropriate amount |

Cucurbitacin B (purity >98%) and S100 (soybean phosphatidylcholine, SPC, Germany LIPOID Company) were dissolved in 1ml ethanol for standby. An HSA solution of 4% (g/ml) was obtained by dissolving the formulation amount of HSA in water and added into the ethanol solution of cucurbitacin B/phospholipid. After being dispersed, the obtained mixed solution was placed in ultrasonic machine and treated by 200W ultrasound at first, followed by dispersion for 5 minutes by 600W ultrasound to obtain emulsion. The organic solvent was removed by rotary evaporation, and then nanoparticles were obtained. The average particle size of the prepared nanoparticles was 156nm, and its particle distribution was narrow so as to pass through $0.45\mu m$, $0.3\mu m$ micropore films. There was no precipitate produced after being left to stand for 60 minutes, thereby indicating that the stability of the nanoparticle system had been greatly improved. After freeze-drying, the system was effectively redispersible and could be returned to the state before freeze-drying. The average particle size after redispersion was 166nm.

Example 3: Preparation of cucurbitacin HSA nanoparticles by dissolution of phospholipid in tert butyl alcohol.

| Cucurbitacin B | 0.01g |
| HSA | 4g |
| S100 | 1.5g |
| Water | appropriate amount |

Cucurbitacin B (purity >98%) and S100 (soybean phosphatidylcholine, SPC Germany LIPOID) were dissolved in 5ml *tert*-butyl alcohol for standby. An HSA solution of 4% (g/ml) was obtained by dissolving the formulation amount of HSA in water and added into the *tert*-butyl alcohol solution of cucurbitacin B/phospholipid. After being dispersed, the obtained mixed solution was placed in an ultrasonic machine and treated by 200W ultrasound at first, followed by dispersion for 5 minutes by 600W ultrasound to obtain emulsion. The average particle size of the prepared nanoparticles was 139nm, and its particle distribution was narrow so as to pass through $0.45\mu m$, $0.3\mu m$ micropore films. There was no precipitate produced after being left to stand for 60 minutes, thereby indicating that the stability of the nanoparticle system had been greatly improved. After freeze-drying, the said system was effectively redispersible and could be returned to the state before freeze-drying. The average particle size after redispersion was 157nm.
Due to the utilization of *tert*-butyl alcohol, the process was simpler without reducing pressure to remove solvent and freeze-drying could be carried out directly.

Example 4: Effect of pressure on particle size of CuB HSA nanoparticles by microfluidic method

| | |
|---|---|
| Cucurbitacin B | 0.01g |
| HSA | 4g |
| S100 | 1.5g |
| Water | appropriate amount |

Cucurbitacin B (purity >98%) and S100 (soybean phosphatidylcholine, SPC Germany LIPOID) were dissolved in 1ml ethanol for standby. An HSA solution of 4% (g/ml) was obtained by dissolving the formulation amount of HSA in water and added into the ethanol solution of cucurbitacin B/phospholipid, and then dispersed followed by homogenization with a microfluidizer to study the effect of pressure and cycles on granularity (Results shown in table 1.)

Table 1 effect of pressure dispersion and cycles on granularity

| pressure ( psi ) | 800 | 8000 | 8000 | 8000 | 8000 | 8000 |
|---|---|---|---|---|---|---|
| cycles | 5 | 2 | 5 | 10 | 15 | 25 |
| particle size ( nm ) | 296 | 96 | 93 | 95 | 98 | 101 |

The data in table 1 showed that the particle size of nanoparticles was obviously reduced as dispersion pressure increased from 800 psi to 8000 psi. Under the pressure of 8000psi, the nanoparticles having particle size less than 100nm could be obtained by cycling twice. However, the particle size was not obviously reduced by increasing number of cycles at 8000 psi, and was even slightly increased by too many cycles.

Example 5: Study on various oil phases

Basic formulation:

| | |
|---|---|
| Cucurbitacin B | 0.05g |
| HSA | 4g |
| S75 | 2g |
| oil | 2g |
| Water | appropriate amount |

The basic formulation further contained phospholipid, the ratio of which to oil was 1 to 1.
Cucurbitacin B (purity >98%), oil and S75 (soybean phosphatidylcholine, SPC Germany LIPOID company) were heated to dissolve, then dispersed in 30ml water followed by being treated in a homogenizer to obtain an average particle size of less than 200nm for standby. An HSA solution of 5% solution was obtained by dissolving the formulation amount of HSA in water and added into the cucurbitacin B emulsion. After a homogeneous blend, the obtained solution was placed in a microfluidizer (cycle once at 10000 psi) for further homogeneous dispersion. The results are shown in table 2.

Table 2 Effect of various oils on granularity

| Categories of oils | MCT | LCT | oleic acid | vitamin E | structure oil | safflower oil |
|---|---|---|---|---|---|---|
| Average particle size ( nm ) | 93 | 103 | 96 | 111 | 96 | 105 |

Nanopaticles having average particle size less than 200 nm could be obtained. The average particle size of nanoparticles prepared from MCT, oleic acid and structure oil was smallest.

Example 6: Effect of the content of MCT on the freeze-thaw stability of CuB HSA nanoparticles

CuB HSA nanoparticles with different content of MCT were prepard according to formulation and process in example 5. The change of particle size was detected after freeze-thaw once (freezing overnight at -30°C, and thawing at room temperature). The results are shown in table 3.

Table 3 Result of freeze-thaw stability test

| Concentration of MCT | 0.5% | 0.75% | 1% | 1.5% | 2% | 3% |
|---|---|---|---|---|---|---|
| Before freezing ( nm ) | 88 | 91 | 95 | 93 | 102 | 98 |
| After freezing ( nm ) | 91 | 98 | 101 | 102 | 116 | 138 |

The data in table 3 showed that the particle size of CuB HSA nanoparicles after freeze-thaw increased greatly as MCT content increased. It should be noted that the particle size of nanoparticles after freeze-thaw increased significantly, especially while the content of MCT in the formulation reached 3%. (The unit of content of MCT was g/ml).

Example 7: Effect of concentration of HSA in water phase on particle size of nanoformulation

Basic formulation:

| | |
|---|---|
| Cucurbitacin B | 0.01g |
| HSA | appropriate amount |
| DLPC | 2g |
| Water | appropriate amount |

The nanoparticles were prepard as described in example 4, wherein the dispersion pressure was selected as 12000 psi and the dispersion was cycled for three times.

When the water phase comprised 1% HSA, the average particle size of nanoparticles was 84.4 nm, and increased to 87.9 nm after filtering through 0.22$\mu$m micropore film; the particle size increased to 246 nm after freeze-thaw.

When the water phase comprised 2% HSA, the average particle size was 91.5 nm, and reduced to 88.8 nm after filtering through 0.22$\mu$m micropore film; the particle size increased to 116 nm after freeze-thaw.

When the water phase comprised 3% HSA, the average particle size was 90.3 nm, and reduced to 90.6 nm after filtering through 0.22$\mu$m micropore film; the particle size increased to 121 nm after freeze-thaw.

When the water phase comprised 4% HSA, the average particle size was 91.8 nm, and increased to 92.2 nm after filtering through 0.22$\mu$m micropore film.

When the water phase comprised 5% HSA, the average particle size was 93.6 nm, and increased to 93.8 nm after filtering through 0.22$\mu$m micropore film.

These indicated that the concentration of HSA had no significant effect on the final particle size of the nanoparticles, but had an effect on the particle size after freeze-thaw. The results of 1%, 2% and 3% HSA are shown in fig. 1.

Example 8: Screening of single cryoprotectant

Basic formulation:

| | |
|---|---|
| Cucurbitacin B | 0.01g |
| HSA | 3g |
| DMPC | 3g |
| Water | appropriate amount |

The nanoformulation was prepared as disclosed in example 4, wherein the dispersion pressure was selected as 12000 psi and the dispersion was cycled for three times. The obtained nanoparticle liquid was freeze-dried according to the following process.

The freeze-drying process was as follows: the nanoparticle liquid was pre-frozen for 6 hours at -70°C, vacuumized until the vacuum degree was below 15 Pa (preferably bellow 5 Pa), then continued being vacuumized for 6 hours at -35°C followed by linear heating from -35°C to 10°C under vacuum for 10 hours, and was stored at 10°C for 2 hours to obtain the desired nanoparticles.

Trehalose, lactose, sucrose, mannitol or glucose was selected respectively as cryoprotectant for cucurbitacin HSA nanoparticles. The test results are shown in the following table, wherein:

1) appearance of freeze-drying preparation: the appearance was marked as (-) when the freeze-dried preparation was full-filled and had smooth surface, a shape of white pie, and in which there was no crystal; the appearance was marked as (+) when the preparation was full-filled and had smooth surface, a shape of white pie, but in which there was small squamous crystal; the appearance was marked as (++) when the preparation was full-filled and had longer needle crystal; the appearance was marked as (+++) when the freeze-dried product had a shape of needle crystal and did not adhere to the bottle wall.

2) transmittance of freeze-dried preparation after hydration: the appearance of the freeze-dried preparation after hydration was marked as (-), when the transmittance after freeze-drying was same as that before freeze-drying (semi-transparence and clear opalescence); and the appearance was marked as (+), when the clarity of the preparation slightly decreased after freeze-drying and the preparation still presented obvious opalescence; the appearance was marked as (++), when the preparation presented almost opaque and slight opalescence. The results are shown in table 4.

Table 4 Screening results of single cryoprotectant

| Cryoprotectant | Appearance of freeze-dried formulation | Duration of redissolution (seconds) | Appearance after hydration |
|---|---|---|---|
| Absent of cryoprotectant | ( + + + ) | 40 | ( + + ) |
| 5% trehalose | ( + + ) | 25 | ( - ) |
| 10% trehalose | ( + + ) | 26 | ( - ) |
| 10% lactose | ( + ) | 28 | ( - ) |
| 5% glucose | ( + + ) | 26 | ( - ) |
| 5% mannitol | ( - ) | 35 | ( + ) |
| 10% sucrose | ( + + ) | 30 | ( - ) |

Obviously, the freeze-thaw stability of nanoparticles with cryoprotectant was better than that without cryoprotectant. The nanoparticles obtained from hydration of the product with cryoprotectant could stabilize for 24 hours, wherein the appearance of preparation with mannitol was best. The duration of hydration of trehalose, lactose, glucose were better than mannitol and sucrose. (The unit of content was g/ml).

Example 9: Study on effect of cryoprotectants in combination

The preparation method of nanoformulation was same as that of example 8. The results are shown in table 5.

Table 5 Effect of cryoprotectants in combination

| Cryoprotectants | Appearance of freeze-dried formulation | Duration of redissolution (seconds) | Appearance after hydration |
|---|---|---|---|
| 10% lactose + 3% sucrose | good | 20 | good |
| 4% mannitol + 8% lactose | good | 20 | good |
| 6% mannitol + 3% lactose | slightly worse | 25 | good |
| 5% mannitol + 5% trehalose | good | 20 | good |

(continued)

| Cryoprotectants | Appearance of freeze-dried formulation | Duration of redissolution (seconds) | Appearance after hydration |
|---|---|---|---|
| 7% mannitol + 3% trehalose | slightly worse | 25 | good |

The data in table 5 showed that combination of cryoprotectants further improved the freeze-thaw stability of nanoparticles so that the nanopaticles were stable within 10 hours. After hydration of freeze-dry products comprising cryoprotectants such as "6% mannitol + 3% lactose" or "7% mannitol + 3% trehalose", the particle size thereof did not change within 48 hours.

Example 10: Docetaxel liposomes

Docetaxel and phospholipid (EPCS, Germany LIPOID company) were weighed with the weight ratio of docetaxel to phospholipid as 1:15, 1:20, 1:25, 1:30 and 1:35 (w/w), respectively. Docetaxel liposomes are prepared according to a typical microfilm method. The results showed that drug crystals precipitated in the liposomes after being placed in refrigerator overnight at $6\pm2°C$ at a docetaxel to phospholipid weight ratio of 1:15 and 1:20; the liposomes aggregated slightly and produced precipitation after being placed in refrigerator overnight at $6\pm2°C$ for 5 days at docetaxel to phospholipid weight ratio of 1:25 and 1:30; the stability of liposomes was good, and no precipitation was produced after the liposomes was placed for 15 days at a docetaxel to phospholipid weight ratio of 1:35.
It had been proved that the amount of docetaxel and phospholipid had a direct effect on the stability of liposomes. When the weight ratio of docetaxel to phospholipid was too big, that would exceed the carrying capacity of the liposome bilayer and result in precipitation, and even could not form stable liposomes. Even though the liposomes had been processed by using freeze-drying, the obtained freeze-dried liposomes were difficult to form those ones before freeze-drying.

Example 11: Illustration of issues in preparation of protein nanoparticles by using chloroform as solvent

Basic formulation:

| | |
|---|---|
| docetaxel | 60mg |
| chloroform | 1ml |
| HSA | 600mg |

Preparation process: the formulation amount of docetaxel was weighed and dissolved in 1ml chroloform for standby. The commercial HSA parenteral solution (parenteral solution of human serum albumin) was diluted with water to obtain 2% solution, i.e. 30ml 2% HSA solution. Chroloform solution of docetaxel was emulsified with 2% HSA solution and dispersed under 20, 000 psi to obtain a solution of nanodispersion. The chroloform was recycled from the obtained solution of nanodispersion under reduced pressure. The residual dispersed liquid was hard to pass through $0.8\mu m$ microporous membrane. After being placed for about 3 hours, the residual dispersed liquid showed a precipitate.

Example 12: Illustration of importance of phospholipid

Formulation:

| | |
|---|---|
| docetaxel | 60mg |
| dehydrated alcohol | 1.5ml |
| soybean phosphatidylcholine | 1500 mg |
| HSA | 600mg |

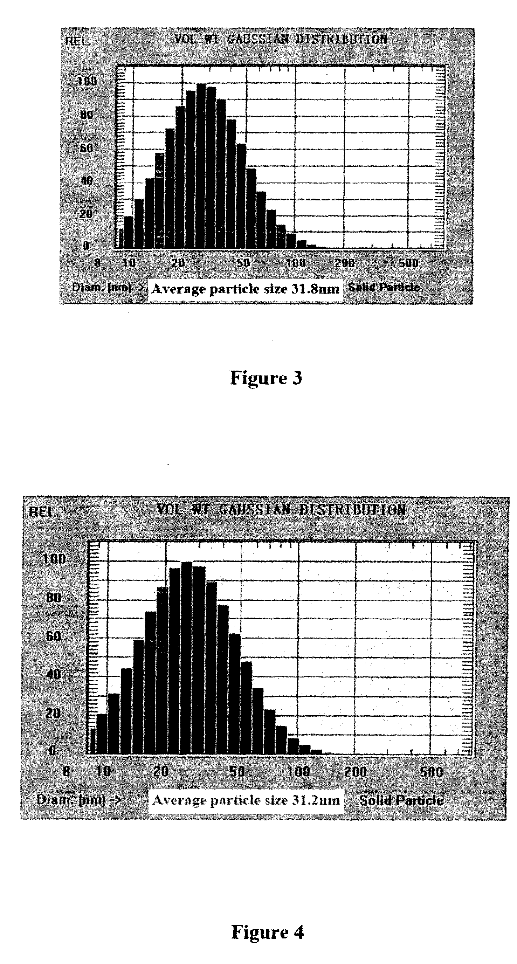

Preparation process: the formulation amounts of docetaxel and phospholipid were weighed and dissolved in 1.5ml dehydrated alcohol to get a drug/phospholipid alcohol solution. HSA was diluted with water to obtain concentration of 2%, i.e. 30ml 2% HSA solution. 2% HSA solution was added into the drug/phospholipid alcohol solution, stirred, and dispersed under 20, 000psi to obtain a translucent dispersion which was easy to pass through 0.22μm microporous membrane to achieve aseptic filtration. The measurements showed that the particle size of the translucent dispersion was 31.8nm (see fig. 3). No precipitation was observed in the translucent dispersion after being placed at room temperature for 30 hours, nor does being placed in refrigerator at 6±2°C for 30 days. The particle size did not change and the average particle size was 31.2 nm.

Example 13: Docetaxel nanoparticle containing phospholipid-protein

Formulation (100mL):

|  |  |
|---|---|
| human serum albumin (HSA) | 2g |
| egg phosphatidylcholine (EPC) | 4g |
| dehydrated alcohol | 3ml |
| docetaxel (Doc) | 0.2g |
| citric acid | appropriate amount |
| distilled water | add to 100ml |

Preparation process:

1. preparation of lipid phase

The formulation amounts of EPC, Doc were precisely weighed and put into a beaker, then 4 ml ethanol (whose concentration is 95% under unspecified circumstances, the same hereinafter) was added. The above substances were dissolved under nitrogen, and mixed to obtain a clear solution, i.e. lipid phase.

2. preparation of water phase

The commercial human serum albumin (20%, W/V (g/ml)) was diluted with sterile injectable water to obtain 4% solution, and adjusted pH to 4 by citric acid to obtain a water phase.

3. dispersion

The water phase was added to the lipid phase, stirred to obtain dispersion liquid of emulsion.

4. homogenization

The dispersion liquid from step 3 was transferred to a microfluidizer to further homogenize for 5 times under 400~12000 psi to obtain a homogeneous liquid.

5. The pH of the homogeneous liquid from step 4 was determined as 5-6. The preparation was obtained after filtration for sterilization with 0.22μm microporous membrane, package and cap. The particle size of the preparation was determined and the average volume diameter was 160 nm.

The stability of the preparation was good. The obtained preparation placed into a refrigerator at 2-8°C for a month had no stratification and sedimentation, and its appearance maintained clear and transparent.

In addition, 10% sucrose could be added to the formulation as required, and pre-freezed at -80°C for 4 hours, vacuumed for 10 hours and kept at 30°C for 5 hours to obtain the freeze-dried formulation.

The obtained freeze-dried formulation could return to the formulation before freeze-drying by being hydrated with added water, and the average volume diameter was 166 nm.

Example 13-1

The formulation and process were the same as those described in example 13, except that egg phosphatidylcholine was replaced by Lipoid E80 (Germany LIPOID Company). The experiment result was similar to that of example 13.

Example 13-2

The formulation and process were the same as those described in example 13, except that egg phosphatidylcholine was replaced by Lipoid S100 (Germany LIPOID Company). The experiment result was similar to that of example 13.

Example 13-3

The formulation and process were the same as those described in example 13, except that egg phosphatidylcholine was replaced by Lipoid EPG (Germany LIPOID Company). The experiment result was similar to that of Example 13.

Example 13-4

The formulation and process were the same as those described in example 13, except that egg phosphatidylcholine was replaced by Lipoid E DPPG (Germany LIPOID Company). The experiment result was similar to that of example 13.

Example 13-5

The formulation and process were the same as those described in example 13, except that egg phosphatidylcholine was replaced by Lipoid E DMPG (Germany LIPOID Company). The experiment result was similar to that of example 13.

Example 13-6

The formulation and process were the same as those described in example 13, except that egg phosphatidylcholine was replaced by Lipoid E DPPC (Germany LIPOID Company). The experiment result was similar to that of example 13.

Example 13-7

The formulation ratio and process were the same as those described in example 13, except that egg phosphatidylcholine was replaced by Lipoid E DMPC (Germany LIPOID Company). The experiment result was similar to that of example 13.

Example 13-8

The process was the same as that described in example 13, except that 0.1g Tween 80 was added to the formulation. The experiment result was similar to that of example 13.

Example 13-9

The process was the same as that described in example 13, except that 0.1g Tween 80 was added to the formulation. The experiment result was similar to that of example 13.

Example 13-10

The process was the same as that described in example 13, except that 0.01g TPGS was added to the formulation. The experiment result was similar to that of example 13.

Example 14

Formulation:

| | |
|---|---|
| 10-hydroxy camptothecin | 10mg |
| DMPC | 100m |
| DMPG | 200mg |
| dehydrated alcohol | appropriate amount |
| human serum albumin (HSA) | 3000mg |

The formulation amounts of 10-hydroxy camptothecin, DMPC and DMPG were weighed and put into a pear-shaped bottle, then an appropriate amount of ethanol was added, and heated to dissolve. The obtained liquid was retrieved under vacuum, and a membranous entity consisting of the drug and phospholipids formed in the bottom. Then 50 ml human serum albumin solution (6% w/v) was added, blended, hydrated to obtain a hydrate. The hydrate was emulsified under 9000-40,000 psi, and cycled for 5 times. The average particle size of obtained formulation was 116 nm. The dispersion can be further freeze-dried to obtain a caked freeze-dried formulation, and the caked freeze-dried formulation could be reconstructed to the original dispersion by adding sterile water or glucose injection or xylitol injection.
It should be noted that the amount, types and ratio of drugs, solvents and proteins used in this example were not limited in any way.

Example 14-1

With regard to camptothecins, such as hydroxy camptothecin, camptothecin, 9-nitrocamptothecin and 7-ethyl-10-hydroxy camptothecin et al., alkaline substances (any alkaline substances, such as sodium hydroxide, sodium carbonate, and lysine et al.) could be added to adjust simply the pH to over 7.4 to dissolve drug and formulate drug solution. Then the drug solution was mixed with phospholipid, and dispersed with homogenizer or ultrasound to reduce the particle size to any desired one (such as less than 100 nm or less than 50 nm). After the phospholipid dispersion containing drugs was obtained, the acidic substance (any acidic substances such as citric acid, lactic acid, fumaric acid, tartaric acid, acetic acid, glucanic acid, lactobionic acid, sorbic acid, ascorbic acid, oxalic acid, formic acid, benzenesulfonic acid, glutamic acid, aspartic acid et al.) was added again to the obtained phospholipid dispersion to adjust the pH to less than 7.4, and then mixed with HSA, dispersed once under high pressure or high-intensity ultrasound to ensure that proteins form disulfide bonds on the surface or internal parts of lipid nanoparticles, and are stably distributed on the nanoparticles. The obtained formulation could be applied by injection, oral and topical administration.
Other operations were the same as those of example 14.

Example 15

Formulation:

| | |
|---|---|
| amphotericin B | 50mg |
| HSPC | 213mg |
| DSPG | 84mg |
| cholesterol (CH) | 52mg |
| methyl-2-pyrrolidone/ methanol | appropriate amount |
| tocopherol | 0.64mg |
| HSA | 400mg |

Preparation process:

The formulation amounts of amphotericin B, HSPC, DSPG, CH, tocopherol were dissolved in a mixed solvent of methyl-2-pyrrolidone and methanol, and then organic solvents were removed under vacuum for standby. 400mg HSA was dissolved in injectable water to obtain 1% (w/v) HSA solution. Amphotericin B mixture was dispersed by HSA solution to obtain dispersion liquid. The dispersion liquid was emulsified at 8000-20,000 psi for at least 3 cycles to obtain translucent dispersion having average diameter of 110 nm. The translucent dispersion could be freeze-dried by using common methods.
The pH value of the translucent dispersion was determined as 5.0-6.0.

Example 16

Formulation:

| | |
|---|---|
| calcitriol | 0.001g |
| DPPC | 0.1g |
| cholesterol | 0.01g |
| HSA | 1g |

The formulation amounts of calcitriol, DPPC, cholesterol were dissolved in 0.5mL methanol for standby. 1% HSA solution was obtained by dissolving the formulation amount of HSA in injectable water and added to the methanol solution of calcitriol, DPPC and cholesterol, then stirred, dispersed to form liposomes which were observed by microscope. The liposomes were granulated by extruder at 60°C, and respectively passed through 0.4, 0.3, 0.22, 0.1, 0.05 and $0.01\mu$m microporous membrane for three times to reduce the particle size of the disperse system to less than 30 nm. The obtained disperse system was further processed in homogenizer to form disulfide bond between the surface thereof and HSA in the internal water phase under the dispersion with the homogenizer, which formed the cross-linked proteins with protective effect on liposomes and ensuring that the liposomes had long-term storage stability. The content of calcitriol in this system was 1mg/100ml, i.e. $10\mu$g/1ml. The injectable water could be further added up to 1000ml, and the drug content was $1\mu$g/1ml. If freeze-dry was needed, 5% glycine (W/V) could be added to the formulation, i.e. 50g glycine was added to 1000ml drug solution, the freeze-drying being performed according to the common methods.

Example 17

Formulation:

| | |
|---|---|
| calcitriol | 0.001g |
| decanoic acid | 1g |
| DLPC | 1g |
| HSA | 10 g |

The formulation amounts of calcitriol, decanoic acid, DLPC were heated to dissolve under nitrogen gas for standby. 3% HSA solution was obtained by dissolving the formulation amount of HSA in injectable water. The lipid phase containing calcitriol was hydrated by the HSA solution, stirred, dispersed to form emulsions observed by microscope. The emulsions were further processed in a homogenizer to form disulfide bond between the surface thereof and HSA under the dispersion with the homogenizer, which formed the cross-linked proteins with protective effect on the emulsions and further ensuring that the emulsions had long-term storage stability. The injectable water could be further added to the emulsions to 1000ml, and the drug content was $1\mu$g/1ml. If freeze-dry was needed, 10% sucrose (g/ml) could be added to the formulation, i.e. 100g sucrose was added to 1000ml drug solution, the freeze-drying being performed according to the common methods.

Example 18

Formulation:

| | |
|---|---|
| amphotericin B | 50mg |
| dimyristoyl phosphatidyl choline ( DMPC ) | 34mg |
| di-octanoyl phosphatidyl glycerol | 60mg |
| citric acid | 5mg |
| human serum albumin | 300mg |

Preparation process: the formulation amounts of amphotericin B, DMPC and di-octanoyl phosphatidyl glycerol were dissolved in an appropriate amount of methanol, and then the methanol was removed under vacuum. 4% HSA solution was obtained by dissolving HSA in distilled water in which citric acid was added. Then the 4% HSA solution was added to the mixture of amphotericin B and phospholipid, hydrated, respectively passed through 0.8, 0.6, 0.4, 0.2, 0.1, 0.05µm microporous membrane at 40°C to obtain dispersion solution. The dispersion solution was treated by ultrasound (i.e. it was dispersed by 600W ultrasound for 1min) in an ultrasonic machine so that disulfide bonds were formed among the proteins.

Citric acid in the formulation could be replaced by any other acids, such as lactic acid, fumaric acid, tartaric acid, acetic acid, gluconic acid, lactobionic acid, sorbic acid, ascorbic acid, oxalic acid, formic acid, benzenesulfonic acid, glutamic acid, aspartic acid et al, and the same result can be obtained.

Example 19

Formulation:

| | |
|---|---|
| propofol | 100mg |
| phosphatidylcholine | 100mg |
| DOPC | 300mg |
| EDTA-2Na | 5mg |
| human serum albumin | 300mg |

Preparation process: The formulation amounts of propofol, phosphatidylcholine, DOPC were heated to dissolve under nitrogen gas for standby. 4% HSA solution was obtained by dissolving the HSA in distilled water in which EDTA-2Na was added. Then the 4% HSA solution was added to the mixture of propofol, and phospholipid, hydrated, respectively passed through 0.8, 0.6, 0.4, 0.2, 0.1, 0.05µm microporous membrane at 40°C to obtain dispersion liquid. The dispersion liquid was treated in an ultrasonic machine, treated by 200W ultrasound fist, then dispersed by 600W ultrasound for 5min to obtain an emulsion having average particle size of 69 nm.

Stability of the emulsion was good. The obtained emulsion placed in a refrigerator (at 2-8°C) for a month had no stratification and sedimentation, and appearance thereof was maintained clear and transparent.

In addition, 0.2% to 20% (W/V) DSPE-PEG could be added to the formulation to obtain PEG-coated protein nanoparticles by using an extra-insertion method.

Example 20: Propofol nanoformulation (100ml)

Formulation:

| | |
|---|---|
| propofol | 1g |
| MCT | 3g |
| SPC | 1g |
| EDTA-2Na | 0.005g |
| human serum albumin (HSA) | 3g |

Preparation process: The formulation amounts of propofol, SPC, MCT were heated to dissolve under nitrogen gas for standby. 5% HSA solution was obtained by dissolving the formulation amounts of human serum albumin (HSA) and EDTA-2Na in injectable water, and then passed through a filter (0.2µm filter) for filtration sterilization and used as water phase. The crude emulsion was obtained by adding the water phase into the oil phase, and then dispersed at 10,000 RPM for 2 minutes. The coarse emulsion was further homogenized under 28,000 psi and repeated twice at 4°C to obtain a terminal emulsion. The terminal emulsion was processed by adding injectable water thereto to achieve the whole volume thereof of 100 ml, and then blended, filtered (by 0.2µm filter), packaged, filled with nitrogen and sealed. The particle size of the emulsion was less than 200 nm.

Example 21

Formulation:

| | |
|---|---|
| propofol | 1g |
| structure oil | 3g |
| DMPG | 5g |
| human serum albumin | 10g |

The preparation process was the same as that of example 20.
In addition, 0.2%~20% (W/V) DPPE-PEG could be added to the formulation to obtain PEG-coated protein nanoparticles by using extra-insertion method.

Example 22

Formulation:

| | |
|---|---|
| propofol | 1g |
| structure oil | 1g |
| TPGS | 5g |
| human serum albumin | 3g |

The preparation process was same as that of example 20.
In addition, 0.2%~20% (W/V) DSPE-PEG can be added to the formulation to obtain PEG-coated protein nanoparticles by using an extra-insertion method.

Example 23

Formulation:

| | |
|---|---|
| entecavir | 0.5g |
| DOPG | 5g |
| HSA | 5g |
| sucrose | 10g |
| water | add to 100ml |

Preparation process: the formulation amounts of entecavir and DOPG were dissolved in a small amount of methanol for standby. 10% HSA and 20% sucrose solution were formulated and added to the above drug phospholipid solution, dispered, homogenized under 4000 ~ 40000psi to obtain liquid having particle size less than 100 nm. Water was added to the obtained liquid so that whole volume was 100 ml and blended to obtain a preparation. The preparation could be prepared into solid by using freezing or spray drying.
Stability of the preparation was good. The obtained preparation placed into a refrigerator (at 2-8°C) for a month had no stratification and sedimentation, and its appearance was maintained translucent.

Example 24: Cucurbitacin E (the purity was greater than 95%)

Formulation (100ml, the concentration of cucurbitacin E was 0.1mg/ml):

| cucurbitacin E | 0.01g |
|---|---|
| oil (MCT) | 1g |
| phospholipid (S75) | 0.1g |
| HSA | 5g |
| mannitol | 6g |
| glucose | 1g |
| water | appropriate amount |

Preparation process: the formulation amounts of cucurbitacin E, MCT and S75 (Lipoid S 75, Shanghai Toshisun Enterprise Co. Ltd.) were heated to dissolve for standby. The formulation amounts of mannitol and glucose were dissolved in 50ml injectable water, and small amounts of activated carbon were added thereto for depyrogenation, cooled, then 25 ml commercial 20% HSA was added, and mixed to obtain a solution comprising HSA. The obtained solution was filtered for sterilization. The obtained aqueous solution comprising HSA was added to oil phase, dispersed, further homogenized under 1000 ~ 20,000 psi, this being repeated for five times to obtain a terminal emulsion. The emulsion was filtered (by 0.2μm filter), packaged, freeze-dried, filled with nitrogen and sealed. Its average particle size was less than 300 nm.

Freeze-drying process: pre-freezed at -74°C for 4h; vacuumized for drying. The emulsion was dried at -30°C for 60min at first stage ; and dried at -20°C for 12h in a second stage, then incubated at 15°C for 5h.

Example 24-1

The other process steps performed were the same as those described in example 24, except that a common amount of antioxidant, such as tocopherol, vitamin C or EDTA-2Na, was added to the formulation.

Example 24-2

The other process steps performed were the same as those described in example 24-1, except that cucurbitacin E in the formulation was replaced by equivalent cucurbitacin extract (comprising commercial cucurbitacin BE raw material), cucurbitacin A, cucurbitacin B, isocucurbitacin B, dihydrocucurbitacin B, cucurbitacin C, cucurbitacin D, isocucurbitacin D, dihydrocucurbitacin D, isocucurbitacin E, dihydrocucurbitacin E, cucurbitacin F, cucurbitacin I, tetrahydrocucurbitacin I or cucurbitacin Q.

Example 25: Cucurbitacin I (the purity was greater than 95%)

Formulation (100ml, the concentration of cucurbitacin I was 0.1mg/ml):

| cucurbitacin I | 0.01g |
|---|---|
| structure oil | 1g |
| phospholipid (S75) | 1g |
| tocopherol | 0.01g |
| HSA | 5g |
| trehalose | 10g |
| water | appropriate amount |

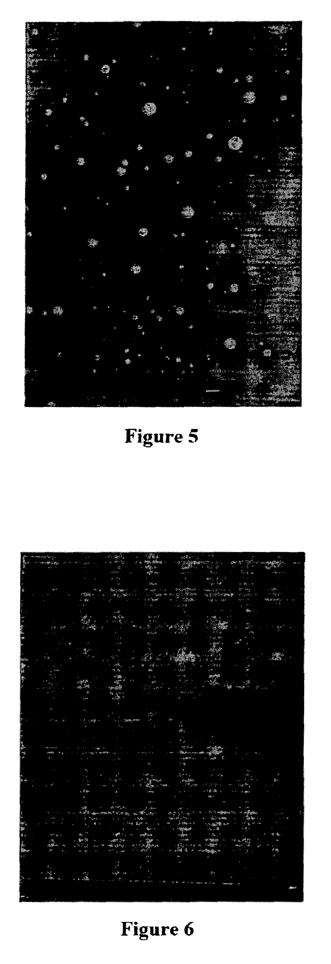

Preparation process: the formulation amounts of cucurbitacin I, structure oil and S75 (Lipoid S 75, Shanghai Toshisun Enterprise Co. Ltd.) and tocopherol were heated to dissolve for standby. The formulation amount of trehalose was dissolved in 50ml injectable water, and small amounts of activated carbon were added thereto for depyrogenation, cooled, then 25 ml commercial 20% HSA was added, and mixed to obtain a solution comprising HSA. The obtained solution was filtered for sterilization. The obtained aqueous solution comprising HSA was added to oil phase, dispersed, further homogenized under 1000 ~ 20,000 psi, this being repeated for three times to obtain a terminal emulsion. The emulsion was filtered (by 0.2μm filter), packaged, freeze-dried, filled with nitrogen and sealed. Its average particle size was less than 200 nm. See fig. 5.

Freeze-drying process: pre-freezed at -74°C for 4h; vacuumized for drying. The emulsion was dried at -30°C for 30min at first stage ; and dried at -20°C for 12h at second stage, then incubated at 15°C for 5h.

The other process steps performed were the same as those described in example 24-1, except that cucurbitacin E in the formulation was replaced by cucurbitacin extract (comprising commercial cucurbitacin BE raw material), cucurbitacin A, cucurbitacin B, isocucurbitacin B, dihydrocucurbitacin B, cucurbitacin C, cucurbitacin D, isocucurbitacin D, dihydrocucurbitacin D, isocucurbitacin E, dihydrocucurbitacin E, cucurbitacin F, cucurbitacin E, tetrahydro-cucurbitacin I or cucurbitacin Q.

Example 25-1

The other process steps performed were the same as described in example 25, except that a common amount of antioxidant, such as tocopherol, vitamin C or EDTA-2Na, was added to the formulation.

Example 25-2

The other process steps performed were the same as those described in example 25-1, except that cucurbitacin I in the formulation was replaced by cucurbitacin extract, cucurbitacin A, cucurbitacin B, isocucurbitacin B, dihydrocucurbitacin B, cucurbitacin C, cucurbitacin D, isocucurbitacin D, dihydrocucurbitacin D, isocucurbitacin E, dihydrocucurbitacin E, cucurbitacin F, cucurbitacin I, tetrahydro-cucurbitacin I or cucurbitacin Q.

Example 26: Cucurbitacin Q (the purity was greater than 95%)

Formulation (100ml, the concentration of cucurbitacin Q was 0.1mg/ml):

| | |
|---|---|
| cucurbitacin E | 0.01g |
| structure oil | 0.1g |
| MCT | 0.4g |
| phospholipid (S75) | 5g |
| HSA | 1g |
| trehalose | 10g |
| xylitol | 2g |
| water | appropriate amount |

Preparation process: The formulation amounts of cucurbitacin Q, structure oil, MCT and S75 (Lipoid S 75, Shanghai Toshisun Enterprise Co. Ltd.) were heated to dissolve for standby. The formulation amounts of trehalose and xylitol were dissolved in 50ml injectable water, and small amounts of activated carbon were added thereto for depyrogenation, cooled, then 5 ml commercial 20% HSA was added, and mixed to obtain a solution comprising HSA. The obtained solution was filtered for sterilization. The obtained aqueous solution comprising HSA was added to oil phase, dispersed, further homogenized under 1000 ~ 20,000 psi, this being repeated twice to obtain a terminal emulsion. The emulsion was filtered (by 0.2μm filter), packaged, freeze-dried, filled with nitrogen and sealed. Its average particle size was less than 200 nm.

Freeze-drying process: pre-freezed at -74°C for 4h; vacuumized for drying. The emulsion was dried at -30°C for 90min at first stage ; and dried at -20°C for 12h at second stage, then incubated at 20°C for 5h.

Example 26-1

The other process steps were the same as those described in example 26, except that common amounts of antioxidant, such as tocopherol, vitamin C or EDTA-2Na, were added to the formulation.

Example 26-2

The other process steps were the same as those described in example 26-1, except that cucurbitacin Q in the formulation was replaced by cucurbitacin extract, cucurbitacin A, cucurbitacin B, isocucurbitacin B, dihydrocucurbitacin B, cucurbitacin C, cucurbitacin D, isocucurbitacin D, dihydrocucurbitacin D, isocucurbitacin E, dihydrocucurbitacin E, cucurbitacin F, cucurbitacin I, tetrahydro-cucurbitacin I, cucurbitacin S or cucurbitacin E.

Example 27 : Desmopressin acetate (and the isoelectric point was 10.9)

Formulation:

| | |
|---|---|
| desmopressin acetate | 400ug |
| dipalmitoyl phosphatidyl glycerol | 1g |
| tetralauroyl diphosphatidylglycerol | 0.1g |
| HSA | 5g |
| trehalose | 10 g |
| water | appropriate amount |

Preparation process: the formulation amounts of dipalmitoyl phosphatidyl glycerol and tetralauroyl diphosphatidylglycerol were dissolved in small amounts of *tert*-butyl alcohol, and then desmopressin acetate aqueous solution was added, dispersed, mixed to form liposomes observed by microscope. The liposomes were adjusted to pH 7.4 by Tris, and then trehalose solution was added, and extruded in turn to pass thin film (Nucleopore) having bilayer 800nm, 300nm, 200nm, 100nm and 50 nm aperture by using extrusion to obtain a phospholipid disperse system for standby. 25 ml commercial 20% HSA solution was added to the said phospholipid disperse system, blended, dispersed at 28,000 psi, this being repeated once to obtain nanoparticles with desired particle size. The average particle size was less than 100nm. The outer layer of liposomes was covered by protein layer.

The formulation could be converted into a solid by using freeze-drying.

Freeze-drying process: pre-freezed at -40°C for 4h; vacuumized for drying. The emulsion was dried at -30°C for 60min at first stage ; and dried at -20°C for 18h at second stage, then incubated at 25°C for 6h.

Example 28

Formulation:

| | |
|---|---|
| octreotide | 1000ug |
| dipalmitoyl phosphatidyl glycerol | 1g |
| tetraoleoyl diphosphatidylglycerol | 0.1g |
| HSA | 5g |
| trehalose | 10 g |
| water | appropriate amount |

Preparation process: the formulation amounts of dipalmitoyl phosphatidyl glycerol and tetraoleoyl diphosphatidylglycerol were dissolved in small amounts of *tert*-butyl alcohol, then desmopressin acetate aqueous solution was added, dispersed, mixed, the pH adjusted to 7.4 by Tris, and the other process steps were the same as those described in example 27.

Example 29

Formulation:

| | |
|---|---|
| zedoary turmeric oil | 1g |
| phospholipid | 2g |
| HSA | 5g |

Prepraration process: zedoary turmeric oil was mixed with phospholipid, then injectable water (50 ml) was added and dispersed to obtain a solution. The particle size of the solution was further reduced to less than 100 nm with a microfluidizer or a homogenizer, and then the solution was filtered for sterilization to obtain a emulsion. Proteins were mixed with sterile injectable water to obtain a 2% solution. 2% protein solution was mixed with the above obtained emulsion, then dispersed with the microfluidizer or homogenizer again, and dispersed only once under 4000psi. Injectable water was added to the obtained solution to achieve a whole volume of 100 ml, then filtered, packaged, and sealed to obtain the desired fomulation whose outer layer was covered by protein layer and the average particle size was less than 150 nm.

Example 30

Formulation:

| | |
|---|---|
| doxorubicin | 0.1g |
| tetraoleoyl diphosphatidylglycerol | 1g |
| DMPC | 5g |
| cholesteryl hemisuccinate | 0.1g |
| ammonium sulfate (150 nm) | 100ml |
| sucrose | 15g |
| human serum albumin | 0.02g |

Preparation process: the formulation amounts of tetraoleoyl diphosphatidylglycerol, DMPC and cholesteryl hemisuccinate were dissolved in ethanol (10ml) to obtain phospholipid ethanol solution for standby. Sucrose (15g) was added to ammonium sulfate (150 nm, 100ml) and dissolved, then commercial 20% human serum albumin (0.1ml) was added, blended, added to the phospholipid ethanol solution, and hydrated to obtain liposomes. The liposomes were extruded in turn by passing through thin film (Nucleopore) having bilayer 800nm, 300nm, 200nm, 100nm and 50 nm aperture with an extruder (Lipex Biomembranes, Inc., Vancouver, BC, Canada), and finally dispersed under 18000psi, repeated twice to obtain nanoparticles with desired particle size. The ammonium sulfate in the water phase outside liposomes was removed by using Sephadex G-50 and microcolumn centrifugal method to obtain liposomes containing proteins. Doxorubicin was dissolved in 0.9% sodium chloride injection to prepare 10mg/ml doxorubicin solution. The doxorubicin solution was blended with the above liposomes, incubated at 50°C for 15 min in a water bath, stirred constantly to obtain liposomal doxorubicin containing protein. Determination of entrapment efficiency showed that, the entrapment efficiency of liposomes prepared by this method was bigger than 90%. The liposomes were filtered with 0.22um filter membrane for sterilization, packaged, and sealed, then storaged at 2 ~ 8°C.
The formulation could be prepared as a solid by using freeze-drying.
Freeze-drying process: pre-freezed at -80°C for 4h; vacuumized at -60°C for drying. Freeze-drying curve: dried at -60°C to -50°C for 2h; at -50°C to -30°C for 10h; at -30°C to 0°C for 6h; at 0°C to 20°C for 4h; heat preservation at 20°C for 2h.
The result of stimulatory and toxicity tests showed that the drug delivery system of the present invention can reduce stimulation of the active drug in vein, phlebitis, venous thrombosis, exosmose and other side effects related to administration.

Example 30-1

The other process steps performed were the same as those described in example 30, except that the drug in the formulation was selected from daunorubicin, epidoxorubicina, vincristine sulphate, mitoxantrone, topotecan hydrochloride, vinorelbine bitartrate or bleomycin. The entrapment efficiencies of prepared liposomes containing protein were all higher than 85%.

Example 31

Formulation:

| doxorubicin | 0.2g |
| cholesterol | 0.2 g |
| tetraoleoyl diphosphatidylglycerol | 1g |
| DMPC | 5g |
| DSPE-PEG2000 | 0.5g |
| ammonium sulfate | 100ml |
| sucrose | 10g |
| human serum albumin | 0.02g |

Preparation process: liposomal doxorubicin containing protein was prepared according to the method described in example 30, then DSPE-PEG2000 (0.5 g) was inserted by using extra-insertion method to obtain PEG-coated Liposomal doxorubicin containing protein. Determination of entrapment efficiency showed that the entrapment efficiency of the obtained liposomes was higher than 90%. The liposomes were filtered with 0.22um filter membrane for sterilization, packaged, and sealed, then stored at 2 ∼ 8°C.

The liposomes could be prepared into solid by using freeze-drying.

Freeze-drying process: pre-freezed at -80°C for 4h; vacuumized at -60°C for drying. Freeze-drying curve: dried at -60°C to -50°C for 1h; at -50°C to -30°C for 8h; at -30°C to 0°C for 6h; at 0°C to 20°C for 2h; heat preservation at 20°C for 2h.

The result showed that the drug delivery system of the present invention could reduce stimulation of the active drug in vein, phlebitis, venous thrombosis, exosmose and other side effects related to administration.

Example 31-1

The other process steps performed were the same as those described in example 31, except that the drug in the formulation was selected from daunorubicin, epidoxorubicina, vincristine sulphate, mitoxantrone, topotecan hydrochloride, vinorelbine or bleomycin. The entrapment efficiencies of prepared liposomes containing protein were all greater than 85%.

Example 32

Formulation:

| doxorubicin | 0.05g |
| tetraoleoyl diphosphatidylglycerol | 0.2g |
| DMPC | 0.3g |
| citric acid (300 mM) | 10ml |
| sucrose | 1g |
| human serum albumin | 0.02g |

Preparation process: the formulation amounts of tetraoleoyl diphosphatidylglycerol and DMPC were dissolved in ethanol (1ml) to obtain phospholipid ethanol solution for standby.

Sucrose (1g) was added to citric acid (300 mM, pH 4.0, 10ml) and dissolved, then commercial 20% human serum albumin (0.1ml) was added, blended, added to the phospholipid ethanol solution, and hydrated, and sonicated by probe to obtain semitransparent liposomal liquid.

Doxorubicin was dissolved in 0.9% sodium chloride injection to prepare 10mg/ml doxorubicin solution. The doxorubicin solution was blended with the above liposomes, the pH adjusted to 7.5 by sodium hydroxide (10mM), incubated at 50°C for 15 min in a water bath, and stirred until liposomal doxorubicin containing protein was obtained. Determination of entrapment efficiency showed that the entrapment efficiency of liposomes prepared by this method was higher than 90%. The liposomes were filtered with 0.22um filter membrane for sterilization, packaged, and sealed, then storaged at 2 ∼ 8°C.

The formulation could be prepared into solid by freeze-drying.

Freeze-drying process: pre-freezed at -80°C for 4h; vacuumized at -60°C for drying. Freeze-drying curve: dried at -60°C to -50°C for 8h; at -50°C to -30°C for 12h; at -30°C to 0°C for 16h; at 0°C to 15°C for 4h; heat preservation at 15°C for 4h.

The result showed that the drug delivery system of the present invention could reduce stimulation of the active drug in vein, phlebitis, venous thrombosis, exosmose and other side effects related to administration.

A photograph of the liposomes is shown in fig. 6.

The pharmacokinetics in mice showed that, $AUC_{0\sim10h}$ of common liposomal doxorubicin was 106mg/L·h, and the $AUC_{0\sim10h}$ of protein-coated liposomal doxorubicin was 330mg/L ·h. Compared with the common liposomal doxorubicin, the protein-coated liposomal doxorubicin could increase the area under the concentration-time curve and extend the duration of drug action.

Example 32-1

The other process steps performed were the same as those described in example 32, except that the drug in the formulation was selected from daunorubicin, epidoxorubicina, vincristine sulphate, mitoxantrone, topotecan hydrochloride, vinorelbine, gemcitabine, huperzine A, bulleyaconitine A or bleomycin. The entrapment efficiency of prepared liposomes containing protein was higher than 85%.

Example 32-2

The other process steps performed were the same as those described in example 32, except that the PEG-coated liposomes containing protein can be prepared by using an extra-insertion method to insert PEG lipid derivatives containing various PEG having different molecular weights into the liposomes.

Example 33

Formulation:

| | |
|---|---|
| elemene | 1g |
| phospholipid | 2g |
| TPGS | 0.5g |
| HSA | 1g |

Preparation process: elemene and phospholipid were mixed with TPGS, then injectable water (50 ml) was added and dispersed to obtain a solution. The particle size of the obtained solution was further reduced to less than 100 nm with a microfluidizer or a homogenizer, and then the solution was filtered for sterilization by passing through 0.22um filter membrane to obtain an emulsion. Proteins were mixed with sterile injectable water to obtain a 2% solution. 2% protein solution was mixed with the above obtained emulsion, then dispersed with the microfluidizer or homogenizer again, and dispersed only once under 2000psi. The addition of surfactant TPGS could reduce the pressure required for dispersion and reduce cost.

Stability of the formulation was good. The obtained formulation placed in a refrigerator (at 2-8°C) for a month had no stratification and sedimentation, and its appearance was still translucent.

The result showed that the drug delivery system of the present invention could reduce stimulation of the active drug in vein, burning sensation and pain during injection, phlebitis, venous thrombosis, exosmose and other side effects related to administration.

Example 34

Formulation:

| | |
|---|---|
| nimodipine | 0.1g |
| SPC | 10g |
| tocopherol hemisuccinate | 0.1g |

(continued)

| | |
|---|---|
| *tert*-butyl alcohol | 20ml |
| HSA | 10g |
| sucrose | 15g |

Preparation process: the formulation amounts of nimodipine, SPC, tocopherol hemisuccinate and *tert*-butyl alcohol were mixed for dissolution, and then 50% sucrose solution (30ml) was added, blended, and 20% HSA solution (20ml) was added and blended to obtain a solution. The solution was homogenized twice under 20,000psi, then the remaining 20% HSA solution (30ml) and injectable water were added to receive a total volume of 250ml, mixed, filtered for sterilization by passing through 0.22um filter membrane to obtain liposomal nanoparticles with a particle size of less than 50 nm.

Example 35: Insertion of ligand containing lipophilic group to nanoparticles

The galactose cholesterol derivative disclosed in patent application CN200610121794.2 of the inventor was inserted by weight of 5% of the phospholipid molar ratio into the nanoparticles of example 25 to obtain the desired nanoparticle. The purpose of active targeted drug delivery could be achieved by inserting transferrin derivatives, folic acid and derivatives, glucosamine derivatives, various kinds of RGD and derivatives thereof, antibodies selected from various murine, humanized, recombinant rat and human antibodies.

Example 36: Alprostadil nanoparticle (5μg/ml)

Formulation:

| | |
|---|---|
| alprostadil | 500μg |
| human serum albumin | 0.03g |
| phospholipid | 3g |
| trehalose | 10g |
| water | appropriate amount (add to 100ml) |

Preparation process: the formulation amounts of alprostadil and phospholipid were dissolved in ethanol (3ml) for standby. The formulation amount of trehalose was dissolved in injectable water (70ml), and small amounts of activated carbon were added for depyrogenation, cooled, then drug phospholipid solution was added, hydrated, dispersed and passed through 0.8, 0.6, 0.4, 0.2, 0.1, 0.05μm microporous membrane to obtain a transparent dispersion solution. Commercial 20%HSA (0.15ml) was added to the dispersion solution, and blended, homogenized under low pressure from 1000 to 10,000 psi for one period, then injectable water was added to receive a total volume of 100ml, blended to obtain final nanoparticles. The final nanoparticles were filtered for sterilization (in 0.2μm filter), packaged, filled with nitrogen and sealed. The average particle size thereof was less than 100 nm. The nanoparticles could be stored in liquid form. If the nanoparticles were needed to be stored in solid form, they could be further processed by freeze-drying, filling with nitrogen, sealing. The freeze-dried formulation was dissolved again in water, and the average particle size thereof was less than 100nm.

Example 36-1: Alprostadil nanoparticle (50μg/ml)

Formulation:

| | |
|---|---|
| alprostadil | 5000μg |
| human serum albumin | 0.3g |
| caprylic acid ethylester | 1g |
| phospholipid | 3g |
| trehalose | 10g |

(continued)

| | |
|---|---|
| water | appropriate amount (add to 100ml) |

Preparation process: the formulation amounts of alprostadil, caprylic acid ethylester and phospholipid were dissolved in dehydrated alcohol (3ml) for standby. The formulation amount of trehalose was dissolved in 70ml injectable water, and small amounts of activated carbon were added thereto for depyrogenation, cooled, then the drug phospholipid solution was added, hydrated, dispersed under 10,000 to 40,000 psi to obtain a dispersion solution having a particle size less than 100nm. Commercial 20%HSA (1.5ml) was added to the dispersion solution, and blended, homogenized under low pressure from 1000 to 10,000 psi for one period, and then injectable water was added to receive a total volume of 100ml, blended to obtain final nanoparticles. The final nanoparticles were filtered for sterilization (in a 0.2$\mu$m filter), packaged, filled with nitrogen and sealed. The average particle size was less than 100nm. The nanoparticles could be stored in liquid form. If the nanoparticles were needed to be stored in solid form, they could be further be processed by freeze-drying, filling with nitrogen, sealing. The freeze-dried formulation was dissolved again in water, and the average particle size thereof was less than 100nm.

Example 37:

100 unit dose of entecavir capsule (0.5mg/capsule)

Formulation:

| | |
|---|---|
| egg albumin | 5 g |
| SPC | 0.1g |
| entecavir | 0.05g |
| lactose | 20g |

Preparation process: the formulation amounts of entecavir and SPC were dissolved in appropriate amount of ethanol for standby. The formulation amount of egg albumin was added to water to prepare a 20% solution, then the 20% solution was added to entecavir/SPC solution, dispersed, homogenized with a homogenizer (400 to 4000 psi) to control the particle size between 500nm to 1000nm, and then lactose was added, and the mixture was spray-dried to obtain protein nanoparticles containing entecavir. 0.1% gum arabic was added, blended and encapsulated (0.5mg/capsule). The obtained protein nanopaticle containing entecavir can also be tabletted by using method of powder being tabletted directly.

Example 37-1

The other process steps performed were the same as those described in example 37, except that entecavir in the formulation was replaced by the following drugs: phenylpropanol (concentration of egg albumin, 10%; egg albumin/phospholipid molar ratio, 300:1), febuprol (concentration of egg albumin, 30%; egg albumin/phospholipid molar ratio, 300:1), vitamin K1 (concentration of egg albumin, 50%; egg albumin/phospholipid molar ratio, 300:1), coenzyme Q10 (concentration of egg albumin, 50%; egg albumin/phospholipid molar ratio, 300:1), cyclovirobuxine D (concentration of egg albumin, 50%; egg albumin/phospholipid molar ratio, 300:1), crataegutt (concentration of egg albumin, 40%; egg albumin/phospholipid molar ratio, 300:1), breviscapinun (concentration of egg albumin, 40%; egg albumin/phospholipid molar ratio, 200:1), flavonihippophae (concentration of egg albumin, 40%; egg albumin/phospholipid molar ratio, 200:1), nimodipine (concentration of egg albumin, 30%; egg albumin/phospholipid molar ratio, 200:1), vitamin D (concentration of egg albumin, 10%; egg albumin/phospholipid molar ratio, 200:1), tanshinone IIA (concentration of egg albumin, 10%; egg albumin/phospholipid molar ratio, 100:1), butylphthalide (concentration of egg albumin, 10%; egg albumin/phospholipid molar ratio, 100:1), ligustilide (concentration of egg albumin, 5%; egg albumin/phospholipid molar ratio, 100:1), felvitene (anethole trithion) (concentration of egg albumin, 10%; egg albumin/phospholipid molar ratio, 100:1), malotilate (concentration of egg albumin, 10%; egg albumin/phospholipid molar ratio, 100:1), norcantharidin acid (concentration of egg albumin, 10%; egg albumin/phospholipid molar ratio, 100:1), cantharidin (concentration of egg albumin, 0.1%; egg albumin/phospholipid molar ratio, 50:1), rubescensine A (concentration of egg albumin, 0.5%; egg albumin/phospholipid molar ratio, 100:1), huperzine A (concentration of egg albumin, 0.05%; egg albumin/phospholipid molar ratio, 1:1), bulleyaconitine A (concentration of egg albumin, 1%; egg albumin/phospholipid molar ratio, 50:1), statin-type hypolipidemic drugs (concentration of egg albumin, 10%; egg albumin/phospholipid molar ratio, 100:1), such as lovastatin, simvastatin, adefovir dipivoxil (concentration of egg albumin, 10%; egg albumin/phospholipid molar ratio, 50: 1), VE nicotinate (concentration of egg albumin, 10%; egg albumin/phospholipid molar ratio, 50:1), zidovudine palmitate (concentration of egg albumin, 10%; egg albumin/phospholipid molar ratio, 50:1), zidovudine myristate (concentration of egg albumin, 10%; egg albumin/phospholipid molar ratio, 150:1), zidovudine brown cholesterol ester (concentration of egg albumin, 10%; egg albumin/phospholipid molar ratio, 150:1), zidovudine cholesterol ester (concentration of egg albumin, 10%; egg albumin/phospholipid molar ratio, 150:1), asarone (concentration of egg albumin, 10%; egg albumin/phospholipid molar ratio, 150:1), fenofibrate (concentration of egg albumin, 10%; egg albumin/phospholipid molar ratio, 150: 1), ursolic acid (concentration of egg albumin, 5%; egg albumin/phospholipid molar ratio, 150:1), 23-hydroxy betulinic acid (concentration of egg albumin, 1%; egg albumin/phospholipid molar ratio, 150:1), 2a,23-dihydroxy ursolic acid (concentration of egg albumin, 10%; egg albumin/phospholipid molar ratio, 150:1), itraconazole (concentration of egg albumin, 10%; egg albumin/phospholipid molar ratio, 150:1), calcitriol (concentration of egg albumin, 0.005%; egg albumin/phospholipid molar ratio, 1:1) or candesartan (concentration of egg albumin, 10%; egg albumin/phospholipid molar ratio, 150:1).

Example 37-2

The other process steps performed were the same as those described in example 37, except that egg albumin in the formulation was replaced by zein, and the drug, SPC and zein were dissolved in appropriate amount of ethanol, and then hydrated with lactose solution and dispersed. The drug was selected from entecavir, phenylpropanol, febuprol, vitamin K1, coenzyme Q10, cyclovirobuxine D, crataegutt, breviscapinun, flavonihippophae, nimodipine, vitamin D, tanshinone IIA, butylphthalide, ligustilide, felvitene (anethole trithion), malotilate, norcantharidin acid, cantharidin, rubescensine A, huperzine A, bulleyaconitine A, statin-type hypolipidemic drugs, such as lovastatin, simvastatin, adefovir dipivoxil, VE nicotinate, zidovudine palmitate, zidovudine myristate, zidovudine brown cholesterol ester, zidovudine cholesterol ester, asarone, fenofibrate, ursolic acid, 23-hydroxy betulinic acid, $2\alpha$,23-dihydroxy ursolic acid, itraconazole or candesartan.

Example 37-3:

| egg albumin | 1g |
| EPC | 10g |
| carotene | 0.05g |
| vitamin E | 0.5g |
| glycerol | 3g |

Preparation process: the formulation amounts of vitamin E, carotene and EPC were dissolved in an appropriate amount of dehydrated alcohol for standby. The formulation amounts of egg albumin and glycerol were mixed with water to obtain 2% solution. The 2% solution was added to vitamin E/carotene/EPC solution, dispersed, homogenized with a homogenizer (4000 to 40000 psi) to control the particle size to less than 500mn, and then distilled water was added to receive a total volume of 100ml, then obtain protein nano-dispersion containing vitamin E and carotene which can be applied externally with the effect of moisturizing and giving delicate skin. Dried Chinese forest frog could be further added to the protein nano-dispersion and then dispered to obtain an external application facial mask. Plant extracts from Ginseng and so on, vitamin C palmitate, coenzyme Q, arbutin, hydroquinone, kojic acid, various kinds of protein hydrolysate and various kinds of amino acids could be further added to the protein nano-dispersion as required.

Example 37-4:

| egg albumin | 10g |
| EPC | 1g |
| vitamin C palmitate | 0.1g |
| vitamin E | 0.1g |
| glycerol | 5g |

Preparation process: the formulation amounts of vitamin E, vitamin C palmitate and EPC were dissolved in appropriate amount of dehydrated alcohol for standby. The formulation amounts of egg albumin and glycerol were mixed with water to obtain a 50% solution. The 50% solution was added to vitamin E/vitamin C palmitate/EPC solution, dispersed, homogenized with a homogenizer (4000 to 40000 psi) to control the particle size to less than 1000nm, and then distilled water was added to receive a total volume of 50ml, then obtain protein nano-dispersion containing vitamin E and vitamin C palmitate which can be applied externally with effect of moisturizing and giving delicate skin. Dried Chinese forest frog could be further added to the protein nano-dispersion, and then dispersed to obtain an external application facial mask. Plant extracts from Ginseng and so on, carotene, coenzyme Q, arbutin, hydroquinone, kojic acid, various kinds of protein hydrolysate and various kinds of amino acids could be further added to the protein nano-dispersion as required.

Example 38: Basic fibroblast growth factor (bFGF) ( isoelectric point was 9.6)

Formulation:

| | |
|---|---|
| bFGF | 500$\mu$g |
| human serum albumin | 10g |
| DMPG | 0.07g |
| 1,2-dimyristoyl-sn-glycero-3-phosphatidic acid | 0.03g |
| trehalose | 10g |
| water | appropriate amount (add to 100ml) |

Preparation process: The formulation amount of BFGF was dissolved by addition of 2 ml water thereto to obtain a solution. The solution was mixed with DMPG, 1,2-dimyristoyl-sn-glycero-3-phosphatidic acid, and dispersed to obtain a bFGF phospholipid solution for standby. The formulation amount of trehalose was dissolved in 30ml injectable water, and then small amounts of active carbon were added for depyrogenation, cooled and HSA (10g) was added and stirred to dissolve, and then mixed with the bFGF phospholipid solution, and passed through 0.8, 0.6, 0.4, 0.2, 0.1, 0.05$\mu$m microporous membranes in turn to obtain a transparent dispersion. Commercial 20% human serum albumin parenteral solution (50ml) was added to the transparent dispersion, blended, homogenized under low pressure from 1000 to 10,000 psi for one cycle, and injectable water was added to receive a total volume of 100ml, blended to obtain a final dispersion. The final dispersion was filtered (with a 0.2$\mu$m filter), packaged, filled with nitrogen and sealed. The average particle size thereof was less than 100nm. The final dispersion could further be freeze-dried by using common methods.

Example 39

Formulation:

| | |
|---|---|
| thymopetidum | 10mg |
| tetramyristoyl diphosphatidylglycerol | 500mg |
| HSA | 1000mg |
| SPC | 100mg |
| lactose | 20g |

Preparation process: the formulation amounts of thymopetidum, tetramyristoyl diphosphatidylglycerol and SPC were dissolved in an appropriate amount of water, stirred, dispersed, homogenized with a homogenizer (under pressure from 4000 to 24000 psi) to obtain thymopetidum dispersion for standby. A 20% solution was obtained by dissolving the formulation amount of HSA in water, and added to the thymopetidum dispersion, dispersed twice under 20000psi to control particle size ranging from 100nm to 200nm, and then lactose was added thereto, spray-dried to obtain protein nanoparticles containing thymopetidum. The nanoparticles could be further processed into capsules, tablets and granules; the nanoparticles could be directly converted into a powder inhalation for pulmonary drug delivery.

Example 39-1

The other process steps performed were the same as those described in example 39, except that DSPE-PEG (molecular weight of PEG ranging from 100 to 10000), (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by usinga post-insertion method.

Example 40

Formulation:

| | |
|---|---|
| human serum albumin (HSA) | 5g |
| diphosphatidylglycerol | 5g |
| methanol | 3ml |
| rubescensine A | 0.2g |
| maltose | 10g |
| Distilled water | add for total volume 100ml |

The other process steps performed were the same as those described in example 13, except that 10% sucrose was replaced by 10% maltose. The average volume particle size of the obtained protein nano-formulation was 110nm.

Example 40-1

The other process steps performed were the same as those described in example 40, except that DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 41

Formulation:

| | |
|---|---|
| human serum albumin (HSA) | 5g |
| diphosphatidylglycerol | 5g |
| DPPE-PEG 1000 | 1g |
| isopropyl ether | 3ml |
| irisquinone | 0.2g |
| trehalose | 10g |
| distilled water | add to a total volume of 100ml |

The other process steps performed were the same as those described in example 13, except that 10% sucrose was replaced by 10% trehalose. The average volume particle size of the obtained protein nano-formulation was 130nm.

Example 41-1

The other process steps performed were the same as those described in example 41, except that DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 42

Formulation:

| | |
|---|---|
| human serum albumin (HSA) | 5g |
| diphosphatidylglycerol | 5g |
| DLPG | 5g |
| DPPE-PEG $_{10000}$ | 0.1g |
| dehydrated alcohol | 3ml |
| homoharringtonine | 0.2g |
| trehalose | 1g |
| lactose | 10g |
| distilled water | add to a total volume of 100ml |

The other process steps performed were the same as those described in example 13, except that 10% sucrose was replaced by 10% lactose and 1% trehalose. The average volume particle size of the obtained protein nano-formulation was 106nm.

Example 42-1

The other process steps performed were the same as those described in example 42, excepting that DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 43

Formulation:

| | |
|---|---|
| human serum albumin (HSA) | 3g |
| SPC | 5g |
| 1,2-dipalmitoyl-sn-glycero-3-phosphatidic acid | 1g |
| MCT | 1g |
| dexamethasone palmitate | 0.02g |
| trehalose | 10g |
| distilled water | add to a total volume of 100ml |

The other process steps performed were the same as those described in example 13, except that 10% sucrose was replaced by 10% trehalose. The average volume particle size of the obtained protein nano-formulation was 90nm.

Example 43-1

The other process steps performed were the same as those described in example 43, except that DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 44

Formulation:

| | |
|---|---|
| human serum albumin (HSA) | 3g |
| SPC | 5g |
| 1,2--di-O-hexadecyl-sn-glycero-3-phosphatidylethanol-amine | 1g |
| MCT | 1g |
| chloramphenicol palmitate | 0.02g |
| distilled water | add to a total volume of 100ml |

The process steps performed were the same as those described in example 13. The average volume particle size of the obtained protein nano-formulation was 150nm.

Example 44-1

The other process steps performed were the same as those described in example 44, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 45

Formulation:

| | |
|---|---|
| human serum albumin (HSA) | 3g |
| dipalmitoyl phosphatidylglycerol | 5g |
| 1,2-di-O-hexadecyl-sn-glycero-3-phosphatidylethanolamine | 1g |
| DMPE-PEG$_{1000}$ | 1g |
| ethanol | 1g |
| gambogic acid | 0.2g |
| maltose | 5g |
| trehalose | 10g |
| distilled water | add to a total volume of 100ml |

The other process steps performed were the same as those described in example 13, except that 10% sucrose was replaced by 10% trehalose and 5% maltose. The average volume particle size of the obtained protein nano-formulation was 170nm.

Example 45-1

The other process steps performed were the same as those described in example 45, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using post-insertion method.

Example 46

Formulation:

| human serum albumin (HSA) | 10g |
| 1,2-dioleoyl-sn-glycero-3-phosphatidylcholine | 3g |
| 1,2-dilinoleoyl-sn-glycero-3-phosphatidylcholine | 1g |
| DMPE-PEG$_{5000}$ | 1g |
| ethanol | 1g |
| dihydroartemisinin | 0.2g |
| maltose | 10g |
| distilled water | add to a total volume of 100ml |

The other process steps performed were the same as those described in example 13, except that 10% sucrose was replaced by 10% maltose. The average volume particle size of the obtained protein nano-formulation was 120nm.

Example 46-1

The other process steps performed were the same as those described in example 46, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using post-insertion method.

Example 47

Formulation:

| human serum albumin (HSA) | 10g |
| lysophosphatidyl inositol | 3g |
| 1,2-dilinolenoyl-sn-glycero-3-phosphatidylcholine | 1g |
| ethanol | 1g |
| dihydroartemisinin | 0.2g |
| trehalose | 10g |
| distilled water | add to a total volume of 100ml |

The other process steps performed were the same as those described in example 13, except that 10% sucrose was replaced by 10% trehalose. The average volume particle size of the obtained protein nano-formulation was 130nm.

Example 47-1

The other process steps performed were the same as those described in example 47, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 48

Formulation:

| Liposoluble extract of toad | 0.1g |
| Lysophosphatidyl inositol | 0.02g |
| lysophosphatidyl serine | 0.2g |
| 1,2-di-O-docosyl-sn-glycero-3-phosphatidic acid | 3g |
| PEG-derivates of porcine serum albumin | 1g |

(continued)

| | |
|---|---|
| trehalose | 10g |
| distilled water | added to a total volume of 100ml |

The process steps performed were the same as those described in example 13. The average volume particle size of the obtained protein nano-formulation was less than 30nm.

Example 48-1

The other process steps performed were the same as those described in example 48, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 49

Formulation:

| | |
|---|---|
| coenzyme Q10 | 0.1g |
| tetramyristoyl diphosphatidylglycerol | 10g |
| bovine serum albumin | 0.1g |
| trehalose | 20g |
| distilled water | add to a total volume of 100ml |

The other process steps performed were the same as those described in example 13, except that human serum albumin was replaced by bovine serum albumin, and 10% sucrose was replaced by 20% trehalose. The average volume particle size of the obtained protein nano-formulation was less than 100nm.

Example 49-1

The other process steps performed were the same as those described in example 49, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 50

Formulation:

| | |
|---|---|
| vitamin $K_1$ | 0.1g |
| 1-palmitoyl-2-oleoyl-sn-glycero-3-phosphatidic acid | 2g |
| bovine serum albumin | 1g |
| trehalose | 10g |
| xylitol | 5g |
| distilled water | add to a total volume of 100ml |

The other process steps performed were the same as those described in example 13, except that human serum albumin was replaced by bovine serum albumin, and 10% sucrose was replaced by 10% trehalose and 5% xylitol. The average volume particle size of the obtained protein nano-formulation was less than 100nm.

Example 50-1

The other process steps performed were the same as those described in example 50, except that 0.01% to 10% (w/v) DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 51

Formulation:

| | |
|---|---|
| 20(S)-protopanoxadiol | 0.1g |
| 1,2-di-O-hexadecyl-sn-glycero-3-phosphatidic acid | 1g |
| 1-tetradecanoyl-2-hydroxy-sn-glycero-3-phosphatidylcholine | 1g |
| 1-linoleoyl-2-hydroxy-sn-glycero-3-phosphatidylcholine | 1g |
| 1,2-dioleoyl-sn-glycero-3-phosphatidic acid | 1g |
| bovine serum albumin | 1g |
| trehalose | 10g |
| sucrose | 5g |
| distilled water | add to a volume of 100ml |

The other process steps performed were the same as those described in example 13, except that 10% sucrose was replaced by 10% trehalose and 5% sucrose. The average volume particle size of the obtained protein nano-formulation was less than 100nm.

Example 51-1

The other process steps performed were the same as those described in example 51, except that DSPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 52

Formulation:

| | |
|---|---|
| 20(R)- ginsenoside Rg3 | 0.1g |
| 1-stearoyl-2-hydroxy-sn-glycero-3-phosphatidylcholine | 1g |
| SPC | 1g |
| HSA | 1g |

The other process steps performed were the same as those described in example 13, except that 10% sucrose was replaced by 10% trehalose. The average volume particle size of the obtained protein nano-formulation was less than 100nm.

Example 52-1

The other process performed was same as those described in example 52, excepting that DSPE-PEG (molecular weight of PEG ranging from 100 to 10000), DMPE-PEG (molecular weight of PEG ranging from 100 to 10000), DPPE-PEG (molecular weight of PEG ranging from 100 to 10000) or DLPE-PEG (molecular weight of PEG ranging from 100 to 10000) was inserted into the above-mentioned formulations by using a post-insertion method.

Example 53

Formulation:

| | |
|---|---|
| ginsenoside $Rg_2$ | 0.1g |
| 1-oleoyl-2-hydroxy-sn-glycero-3-phosphatidylcholine | 0.01g |
| EPG | 1g |
| EPC | 10g |
| 1,2-distearoyl-sn-glycero-3-phosphatidic acid | 0.1g |
| 1,2-ditetradecanoyl-sn-glycero-3-phosphatidyl-rac-glycerol | 0.1g |
| 1,2-distearoyl-sn-glycero-3-phosphatidyl-rac-glycerol | 0.1g |
| dipalmitoyl phosphatidylcholine | 0.1g |
| HSA | 0.1g |

The other process steps performed were the same as those described in example 13, except that 10% sucrose was replaced by 10% trehalose. The average volume particle size of the obtained protein nano-formulation was less than 100nm.

Example 53-1

The other process steps performed were the same as those described in example 53, except that DSPE-PEG (molecular weight of PEG ranging from 100 to 10000), PEG-THS, PEG-CHS, PEG-CHM, DMPE-PEG (molecular weight of PEG ranging from 100 to 10000), DPPE-PEG (molecular weight of PEG ranging from 100 to 10000) or DLPE-PEG (molecular weight of PEG ranging from 100 to 10000) was inserted into the above-mentioned formulations by using a post-insertion method.

Example 54

Formulation:

| | |
|---|---|
| aescine sodium | 500mg |
| 2-O-octacosyl-sn-glycero-3-phosphatidic acid | 1g |
| 1,2-dipalmitoyl-sn-glycero-3-phosphatidyl-rac-glycerol | 2g |
| porcine serum albumin | 10g |
| water | appropriate amount |

Preparation process: aescine sodium, 2-O-octacosyl-sn-glycero-3-phosphatidic acid, 1,2-dipalmitoyl-sn-glycero-3-phosphatidyl-rac-glycerol and porcine serum albumin were dispersed in water, stirred, homogenized with a homogenizer under 800psi, and then water was added to reach a final volume of 50ml, and homogeneously blended to obtain a formulation. The formulation can be applied externally for detumescence.

Example 54-1

The other process steps performed were the same as those described in example 54, except that DSPE-PEG (molecular weight of PEG ranging from 100 to 10000), DPPE-PEG (molecular weight of PEG ranging from 100 to 10000), DMPE-PEG (molecular weight of PEG ranging from 100 to 10000) or DLPE-PEG (molecular weight of PEG ranging from 100 to 10000) was inserted into the above-mentioned formulations by using a post-insertion method.

Example 55: Azithromycin ophthalmic solution (100ml)

Formulation:

| | |
|---|---|
| azithromycin | 1g |
| DLPG | 2g |
| EDTA-2Na | 0.002g |
| benzalkonium | 0.003g |
| HSA | 1g |
| water | appropriate amount |

Preparation process: a first solution was obtained by dissolving azithromycin and DLPG in ethanol; EDTA-2Na and HSA were dissolved in water to formulate a 2% solution. The 2% solution was added to the first solution and stirred to obtain a mixture. The pH of the mixture was adjusted to 6 to 8 by using citric acid, then microfluidic dispersed, and treated twice under 12000 psi. Then benzalkonium and water were added to adjust the total volume to 100ml, and blended to obtain azithromycin ophthalmic solution.

Example 56:

Formulation:

| | |
|---|---|
| miconazole nitrate | 1g |
| didecanoyl phosphatidylcholine | 10g |
| HSA | 1g |
| chitosan | 1g |
| water | appropriate amount |

Preparation process: miconazole nitrate and didecanoyl phosphatidylcholine were dissolved in an appropriate amount of ethanol for standby; chitosan were dissolved in appropriate amount of acetic acid, and then commercial 20% HSA (5ml) and water (50ml) were added and blended, added to miconazole nitrate phospholipid solution, dispersed, treated for three times under 16000 psi, adjusted to pH 4 ~ 5, adjusted the total volume to 100ml, and blended to obtain miconazole nitrate nanoparticle formulation. The formulation could be administered externally to the vagina.

The chitosan could be replaced by octadecylamine, fatty amine, polyamine cholesterol cationic lipid and derivative of cholesterol cationic lipid to ensure that the nanoparticles are positively charged to achieve the purpose of vaginal adhesive administration.

Example 57

Formulation:

| | |
|---|---|
| simvastatin | 1g |
| phospholipid | 2g |
| egg albumin | 2g |
| lactose | 10g |
| water | appropriate amount |

Preparation process: the formulation amounts of simvastatin and phospholipid were dissolved in an appropriate amount of ethanol for standby. A 5% solution was obtained by dissolving the formulation amounts of egg albumin and lactose in water, added to simvastatin/phospholipid solution, dispersed, homogenized with a homogenizer (under 100

~ 10000 psi) to control the particle size ranging from 300nm to 500nm, and freeze-dried or spray-dried to obtain protein nanoparticles containing simvastatin which could be capsulated, directly compressed or formulated into granules.

Example 57-1

The process steps performed were the same as described in example 57, except that the process was used to prepare formulation of statins for oral administration, articular cavity or subcutaneous injection.

Example 58

Formulation:

| | |
|---|---|
| calcitonin | 1g |
| phospholipid | 2g |
| protein | 1g |
| trehalose | 2g |

The preparation process was the same as that disclosed in example 57. The obtained formulation could be administered intranasally or by an injection route.

Example 59

Formulation:

| | |
|---|---|
| lumbrokinase | 1g |
| phospholipid | 2g |
| protein | 2g |
| mannitol | 10g |
| water | appropriate amount |

The preparation process was the same as that disclosed in example 57. The obtained formulation can be administered via the oral route.

Example 60: Nano silver

Formulation:

| | |
|---|---|
| silver nitrate | 1g |
| hydrazine hydrate | 1g |
| phospholipid | 5g |
| PEG porcine serum albumin | 1g |
| water | added to a total volume of 100ml |

Preparation process: silver nitrate was dissolved in water to prepare a 0.1Mol/L solution, and then phospholipid was added, dispersed for three times under 20000psi for standby. PEG porcine serum albumin was dissolved in water to prepare a 5% solution. The 5% solution was added to the above liposome dispersion, dispered once under 10000 psi and subjected to ultrafiltration to remove unenveloped silver nitrate, then hydrazine hydrate was added to the remaining liposome dispersion, stirred and reacted for 6 hours, subjected to ultrafiltration to remove redundant hydrazine hydrate, and finally a nano silver colloid with average particle size less than 100nm was obtained by addition of water to adjust the total volume to 50ml and utilization of acids to adjust the pH to 6 ~ 7. The nano silver colloid can be administrated via an external (including cavity administration such as vaginal, rectal drug administration), oral or injection route.

Example 60-1

The process steps performed were the same as described in example 60, except that the process was used to prepare a colloid dispersion system of nano gold, nano iron and so on. The colloid dispersion system can be administered via an external (including cavity administration such as vaginal, rectal drug administration), oral or injection route or used for magnetic targeted drug delivery.

Example 61: Cucurbitacin I (the purity was larger than 98%)

Formulation (100ml, the concentration of cucurbitacin I was 0.1mg/ml):

| | |
|---|---|
| cucurbitacin I | 0.01g |
| DMPC | 5g |
| DSPE-PEG2000 | 0.2g |
| HSA | 0.1g |
| trehalose | 10g |
| water | appropriate amount |

Preparation process: the formulation amounts of cucurbitacin I and DMPC were dissolved in small amounts ethanol for standby. The formulation amount of trehalose was dissolved in 70ml injectable water, and then a small amount of activated carbon was added for depyrogenation, followed by filtration, and the filtrate was cooled, added to the cucurbitacin I phospholipid solution, dispersed, passed through 0.8, 0.6, 0.4, 0.2, 0.1, 0.05$\mu$m microporous membranes in turn to obtain a transparent dispersion for standby; 25ml commercial 20% HSA was added, blended and filtered for sterilization to obtain an aqueous solution containing HSA. The aqueous solution containing HSA was added to the above disperse system, homogenized twice under high pressure from 1000 to 20,000 psi to obtain a dispersion of nanoparticles DSPE-PEG$_{2000}$ (0.2g) was inserted into the dispersion nanoparticle by using an extra-insertion method, filtered (with a 0.2$\mu$m filter) for sterilization, packaged and sealed to obtain a formulation.
The obtained formulation could be treated by using a freeze-drying method.
Freeze-drying process: pre-freezed at -74°C for 4h; vacuumized for drying. The formulation was dried at -30°C for 30min at first stage ; and dried at -20°C for 12h at second stage, then incubated at 15°C for 5h.

Example 61-1

The other process steps performed were the same as those described in example 61, except that a regular amount of antioxidant, such as tocopherol, vitamin C or EDTA-2Na, was added to the formulation.

Example 61-2

The other process steps performed were the same as those described in example 61, except that cucurbitacin I in the formulation was replaeed by cucurbitacin extract (including commercial cucurbitacin BE), cucurbitacin A, cucurbitacin B, isocucurbitacin B, dihydrocucurbitacin B, cucurbitacin C, cucurbitacin D, isocucurbitacin D, dihydrocucurbitacin D, isocucurbitacin E, dihydrocucurbitacin E, cucurbitacin F, cucurbitacin E, tetrahydro-cucurbitacin I or cucurbitacin Q.

Example 61-3

The other process steps performed were the same as those described in example 61, except that the DPPE-PEG, DMPE-PEG, DLPE-PEG containing various PEG having different molecular weights were inserted into the nanoparticle by using an extra-insertion method.

Example 62:

Formulation:

| | |
|---|---|
| cucurbitacin Q | 0.1g |
| polyene phosphatidylcholine | 10g |
| *tert*-butyl alcohol | 20ml |
| protein | 1g |
| sucrose | 20g |

Preparation process: the formulation amounts of cucurbitacin Q, polyene phosphatidylcholine and *tert*-butyl alcohol were stirred to ensure dissolution, and then 40 ml 50% sucrose solution was added, mixed, and 5ml 20% HSA solution was added and mixed to obtain a mixture. The mixture was homogenized once under high pressure of 20000psi, and injectable water was added to achieve a total volume of 100ml, blended, filtered by 0.22um filtration membrane for sterilization to obtain the desired formulation having an average particle size less than 50 nm. The formulation was packaged in 1ml of each ampule, and freeze-dried by using a common method to obtain 1000µg/ml/ampule of high concentration freeze-dried products. The high concentration formulation could be obtained by adopting the method of the present invention for the reason that cucurbitacin Q had potent pharmacological activity and daily dose thereof was less than 1000µg. As a result, cost of production, transportation, storage and administration could be reduced.

Example 63: Accelerated test on chemical stability of docetaxel nanoparticle

Taking the formulation of example 13 as an example, an accelerated test was performed to detect the freeze-dried formulaltion from example 13.

Test conditions:

Chromatographic column: Diamond ODS column (4.6 mm×200 mm, 5 µm); mobile phase: acetonitrile-water (50:50, V:V); column temperature: room temperature; detection wavelength: 230 nm, number of theoretical plates was not less than 5000; drug concentration: about 20µg/ml; injection volume: 20 µL; external standard method.
The results were shown in table 6.

Table 6 Result of assav

| Time (month) | Appearance | Relative content (%) | Impurity | Entrapment efficiency (%) |
|---|---|---|---|---|
| 0 | off-white color | 101.3 | 1.26 | 93.2 |
| 3 | off-white color | 100.8 | 1.22 | 94.1 |
| 6 | off-white color | 101.1 | 1.18 | 94.6 |
| 12 | off-white color | 100.6 | 1.32 | 94.5 |

The particle size did not change obviously.

Example 64: Acute toxicity tests on various docetaxel formulations

Drugs: formulation from example 13 and docetaxel tween 80 solution prepared by using the formulation and preparation process as those of the commercial docetaxel formulation.

Animals : male mice (18g-20g)
Dose: 100mg/kg.
Administration route: intravenous injection.
Observation period: 7 days.
The test was repeated three times.
Survival of mice is shown in table 7.

Table 7 Survival of mice treated by various formulations

| formulations | dosage | survival/total number |
|---|---|---|
| example 13 | 100mg/kg | 10/10 , 9/10 , 9/10 |
| docetaxel tween 80 solution | 100mg/kg | 5/10 , 3/10 , 6/10 |

The three batches of data showed that the formulation of the present invention had much lower toxicity than docetaxel tween 80 solution prepared by using the formulation and preparation process of the commercial docetaxel formulation.

Example 65: Paclitaxel nanoparticle

The other process steps performed were the same as those described in example 13, except that docetaxel was replaced by paclitaxel.

The obtained dispersion easily passed through a $0.22\mu m$ microporous membrane for aseptic filtration. There was no precipitation produced in the dispersion left to stand for about 30 hours.

| control example of example 65 | illustration of the issue in preparation of protein nanoparticle by using chloroform |
|---|---|
| paclitaxel | 60mg |
| chloroform | 1ml |
| HSA | 600mg |

Preparation process: the formulation amount of paclitaxel was dissolved in 1ml chloroform to obtain paclitaxel chloroform solution for standby. Commercial HSA parenteral solution was diluted in water to formulate 2% solution, i.e. 30ml 2% HSA solution. The paclitaxel chloroform solution was emulsified with the 2% HSA solution, and dispersed at 20,000 psi to obtain a nano-dispersion solution. The nano-dispersion solution was concentrated under reduced pressure to recycle chloroform, and the remaining liquid was hard to pass through a $0.8\mu m$ microporous membrane, and precipitated after being left to stand for 1 hour.

Example 66: Toxicity test for paclitaxel nanoparticle

Drugs: formulation from example 65 and paclitaxel solution prepared by using the formulation and preparation process of the commercial paclitaxel formulation (adopting Cremophor RH40 polyethylene glycol hydrogenated castor oil).

Animals : male mice (18g-20g)
Dose: 50mg/kg.
Administration route: intravenous injection.
Observation period: 7 days.
The test was repeated three times.
Survival of mice is shown in table 8.

Table 8 Survival of mice treated by various formulations

| formulation | dose | survival/total number |
|---|---|---|
| example 65 | 50mg/kg | 10/10 , 10/10 , 10/10 |
| paclitaxel solution | 50mg/kg | 4/10 , 5/10 , 4/10 |

The three batches of data showed that, the formulation of the present invention had much lower toxicity than paclitaxel solution prepared by using the formulation and preparation process of the commercial paclitaxel formulation.

Example 67: Efficacy test of cucurbitacin B phospholipoprotein nanoformulation

Microplate Reader (Switzerland Sunrise); 3-(4,5-dimethylthiazol-2-yl)-2, 5-diphenyl-2H-tetrazolium bromide (MTT) (America Fluka); DMSO (Sigma); RPMI 1640 media (GIBCO); fetal bovine serum (FBS) (Tian Jin TBD Center for Biotechnology Development).

Studies were respectively carried out on the inhibition effect of cucurbitacin B HSA nanoparticle on the growth of tumor cells in vitro in human laryngocarcinoma epithelial cells (Hep-2), human hepatoma carcinoma cell (HepG-2), human adenocarcinoma of lung cell (A549), and inhibition effect of the same dosage of cucurbitacin B emulsion and liposomes in these cells were compared.

1. method

Cells were cultured in RPMI 1640 media containing 10% FBS in an incubator at 37°C and 5%$CO_2$, and passaged every 3 to 4 days. Cells in exponential growth phase were digested by 0.25% pancreatin containing 1mM EDTA, and then suspended in culture media to achieve a cell concentration of $2.5\times10^4$/ml, seeded in 96-well plate with three re-holes in 100$\mu$L per well. The blank wells (blank) were provided with culture medium. After the cells were cultured for 18-24h and adhered, they were divided into different groups to which various concentrations of cucurbitacin B were added, and one as control group was lacking cucurbitacin B. After the cells were cultured for a certain time (for example 24 hours or 48 hours), 10$\mu$L 5 mg/ml MTT reagent was added to each well, cultured for 4 hours, and then 100$\mu$l 10%SDS (containing 10mM hydrochloric acid) was added and cultured overnight in an incubator. OD (optical density) value of each well was determined in the microplate reader (at 570nm wavelength). Cell inhibition rate was calculated according to OD value, and the following formula was used: cell inhibition rate (IR)=[(mean OD value of control group - mean OD value of blank group)-(mean OD value of test groups - mean OD value of blank group)]/( mean OD value of control group - mean OD value of blank group).

i.e., cell inhibition rate (%) = 1-(OD test - OD blank)/( OD control - OD blank).

(1) inhibition effect of cucurbitacin B solution on the growth of Hep-2 cells

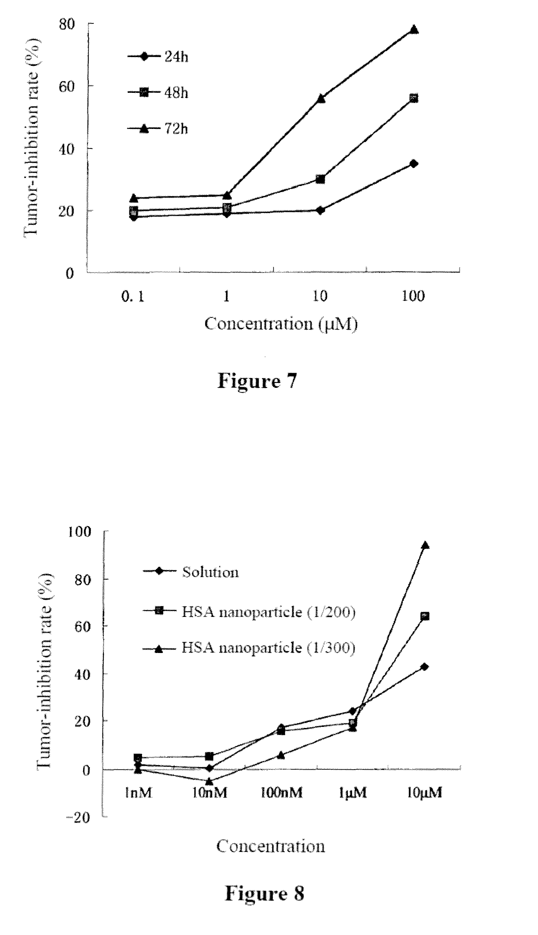

Concentration of 0.1, 1, 10 and 100$\mu$M of cucurbitacin B (dissolved in DMSO) were added respectively to the culture medium of Hep-2 cells to detect inhibition effect of cucurbitacin B on the growth of Hep-2 cells after culture for different times. It was shown that inhibition effect of cucurbitacin B on the growth of Hep-2 cells enhanced along with the extended time and increased dose. In particular, this tendency was more obvious at the present of long action time and high dose. The results were shown in figure 7.

(B) inhibition effect of nanoparticle prepared from various ratio of cucurbitacin B/ HSA (human serum albumin) on the growth of various tumor cells.

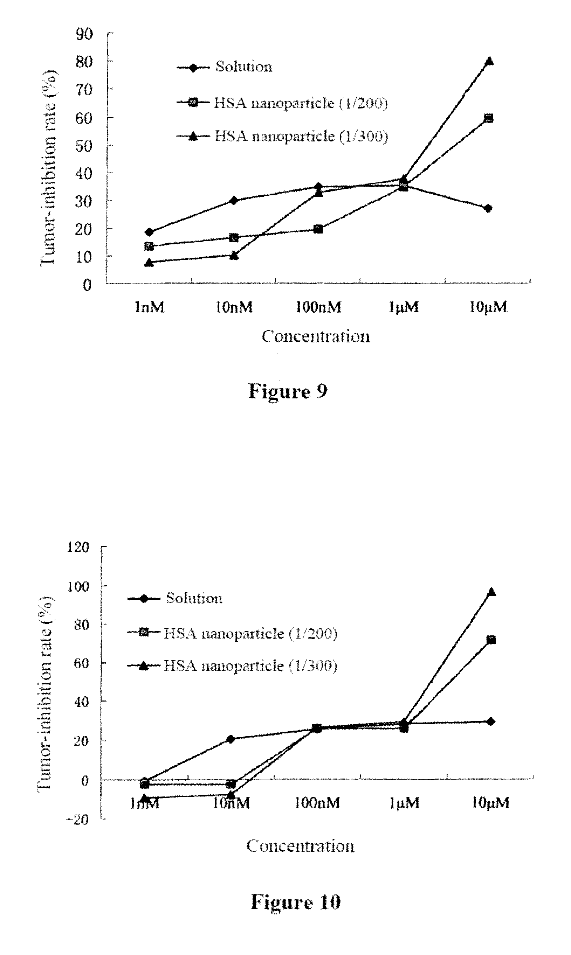

Cucurbitacin B HSA nanoparticles containing 2% or 3% HAS (in which all the concentrations of CuB were 0.1mg/ml) were prepared as described in example 4. The ratio of cucurbitacin B and HSA in the formulation of 2%HSA was 1/200 (w/w), and that in the formulation of 3%HSA was 1/300 (w/w). In order to illustrate the importance of preparation of nanoparticle, we prepared CuB solution by dissolving CuB in injectable water containing 10% ethanol, and added HSA to the CuB solution on the ratio of CuB/HSA of 1/300 (w/w) to get a concentration of 3% for HSA in CuB/HAS solution. Studies were respectively performed on inhibition effects of cucurbitacin B HSA nanoparticle and CuB/HSA solution on the growth of Hep-2, A549 and HepG-2 tumor cells at different doses. The results were shown respectively in figures 8, 9 and 10.

The figures showed that inhibition effect of cucurbitacin B nanoparticle on the growth of Hep-2, A549 and HepG-2 cells was enhanced along with the increased dose. The inhibition effect of cucurbitacin B HSA solution on Hep-2 cells was enhanced gradually along with the increased dose; however the inhibition effect was not enhanced obviously after the dose was greater than 10nM in HepG-2 cells; and the inhibition effect decreased when the dose was greater than 100nM in A549 cells. The inhibition effects of cucurbitacin B HSA nanoparticles in each of the types of cell were significantly greater than that of cucurbitacin B HSA solution at a dose of 1$\mu$M, wherein the inhibition effect of 3% HSA nanoparticle was greater than that of a 2% HSA nanoparticle. It was found that the change of inhibition effect of CuB HSA solution on several tumor cells along with increased dose was not obvious as that of nanoparticles, and the inhibition effect decreased after the dose was greater than 100nM in A549 cells, probably because the uptake mechanism of A549 cells was saturated by large amount of HSA not carrying drug, and hindered the drug transport into this cell. With regard to the common liposome and emulsion, they could only pass through the cell membrane via non-specific fusion or endocytosis and so on, and the affinity and selectivity of uptake of this cell to them were lower than those of HSA nanoparticle. The advantage was more obvious when the concentration HSA in nanoparticle was 3%.

Example 68: Detection of inhibition effect of cucurbitacin B HSA nanoparticle, emulsion and liposome on growth of various tumor cells

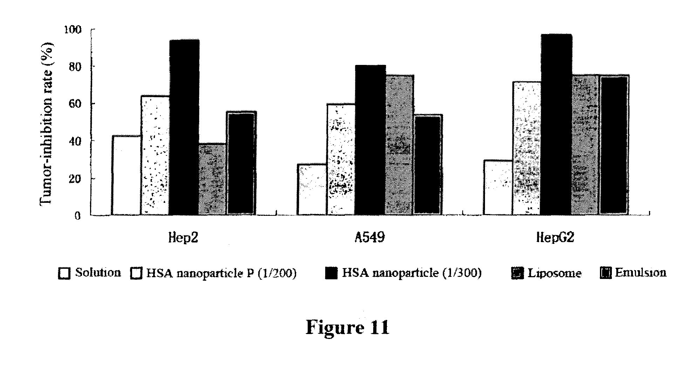

Inhibition effects of various formulations of CuB (cucurbitacin B/HSA solution, CuB HSA nanoparticle, CuB liposome and CuB emulsion) on Hep-2, A549 and HepG-2 tumor cells were detected. The results were shown in figure 11.

As shown in figure 11, 3%HSA CuB nanoparticle showed the most potent inhibition effect on Hep-2, A549 and HepG-2 cells; and 2% HSA CuB nanoparticle was stronger than those of liposome and emulsion on Hep-2 cell, and lower than that of liposome on A549 cell, similar to those of emulsion and liposome on HepG-2 cell. The inhibition effects of CuB HSA solution in each group were weak, but stonger than liposome in Hep-2 cell.

The above results showed that CuB HSA nanoparticle had a strong inhibition effect on the growth of tumor cells. HSA could bind the albumin binding protein (ABP) of tumor cell membrane so that the drug carried by it could be easily taken in by tumor cells. In CuB HSA nanoparticle, the drug was mainly packed in particles formed by protein, and was transported into cells through HSA on the surface of particle binding with cell surface receptors; as for CuB HSA solution, only one portion of CuB formed molecular binder with HAS, and the majot portion of drug and HSA existed in the solution in free molecular state.

Example 69: Effect of anti-tumor and leucocyte-promoting of cucurbitacin B HSA nanoparticle in vivo

Transplanted liver cancer (H22) model in nude mice were established for antitumor drug tests. The nude mice were randomly divided into six groups (10 animals for each group): blank control group, cyclophosphamide positive control group (CTX 20mg·kg$^{-1}$), treatment groups (high, medium and low dosage, 0.20mg·kg$^{-1}$, 0.10mg·kg$^{-1}$ and 0.05mg·kg$^{-1}$) and solution group (non-nanoparticle formulation). The negative control group received injection of physiological saline. After inoculation for four days and with the tumors grown to a certain volume, the mice were treated on the 5th, 7th, 9th, 11th and 13th day. On the 14th day, the mice were sacrificed by cervical dislocation and weighed, dissected to peel off the tumor tissue which was weighed by an electronic balance. The tumor-inhibition rates were calculated by using the following formula:

$$\text{tumor-inhibition rate (\%)} = (1 - \text{average tumor weight of treatment group} / \text{average tumor weight of control group}) \times 100\%,$$

all the data were expressed as mean $\pm$ standard deviation (i.e. $\bar{x} \pm$SD);
the mice were bled at caudal vein for routine white blood cell count.

The results were shown in table 9.

Table 9 Anti-H22 effect of cucurbitacin B nanoparticle

| groups | dosage | Number of mice (begin/ end) | Body weight (g) | | Weight of tumor (g) ($\bar{x} \pm$SD) | Tumor-inhibition rate(%) |
|---|---|---|---|---|---|---|
| | | | begin | end | | |
| blank group | - | 10/8 | 19.36 | 25.86 | 2.82±0.71 | - |
| CTX | 20mg/kg | 10/9 | 19.77 | 21.08 | 1.26±0.63** | 55.3 |
| High dosage | 0.2mg/kg | 10/10 | 20.06 | 24.16 | 1.18±0.58** | 58.2 |
| Medium dosage | 0.1mg/kg | 10/10 | 19.62 | 25.38 | 1.43±0.67** | 49.3 |
| Low dosage | 0.05mg/kg | 10/10 | 19.88 | 25.66 | 1.77±0.55* | 37.2 |
| Drug solution | 0.1mg/kg | 10/8 | 19.62 | 24.71 | 1.91±0.83* | 32.3 |
| note : **P<0.01 *P<0.05 | | | | | | |

Two mice died in the cucurbitacin B solution group, and none died in the nanoparticle group, thus the HSA nanoparticle formulation had lower toxicity than the solution, and tumor inhibition rate of the HSA nanoparticle formulation was greater than the solution. When the dosage of the HSA nanoparticle was half of that of the solution, the HSA nanoparticle still had higher tumor inhibition rate than the solution; when the dosage was onefold of the solution, the HSA nanoparticle still had lower toxicity than the solution, thus had an improvement in efficacy and toxicity reduction.

The leukocyte number of negative control group, cyclophosphamide positive control group (CTX ,20mg·kg$^{-1}$),

treatment group (high, medium and low dosage, 0.20mg·kg$^{-1}$, 0.10mg·kg$^{-1}$ and 0.05mg·kg$^{-1}$) and solution group respectively were 6.89, 3.11, 8.63, 8.11, 8.21 and 8.26($\times 10^9 \cdot$L$^{-1}$). The leukocyte number of cyclophosphamide positive control group was greatly reduced. The leukocyte number of all cucurbitacin groups was greater than that of cyclophosphamide positive control group, and greater than that of the blank control group.

## Claims

1. A drug delivery system, **characterized by** comprising a protein-phospholipid dispersion, in which the weight ratio of protein to phospholipid is 1:300 to 300:1, and the particle size is between 5nm and 1000nm.

2. The drug delivery system according to claim 1, wherein, in the protein-phospholipid dispersion, the surface of phospholipids or the surface and interior of phospholipids are coated by protein layer, or the inner and outer parts of the liposome bilayer are coated by protein, or protein nanoparticles are formed in water phase of the liposomes.

3. The drug delivery system according to claim 1 or 2, wherein the protein is selected from at least one of human albumin, bovine serum albumin, egg albumin, zein, hemoglobin and derivatives thereof, galactose glycated albumin and modified porcine seralbumin.

4. The drug delivery system according to any one of claims 1 to 3, wherein, the phospholipids are selected from at least one of soybean lecithin, egg yolk phosphatidylcholine (ovolecithin), EPG, diphosphatidylglycerol, sphingomyelin, phosphatidyl serine, phosphatidylinositol, phosphatidylethanolamine, hydrogenated soybean phosphatidylcholine, hydrogenated egg yolk phosphatidylcholine, distearoyl phosphatidylcholine, dipalmitoyl phosphatidylcholine, dioleoyl phosphatidylcholine, dimyristoyl phosphatidyl choline, dilauroyl phosphatidylcholine, dicaprinoyl phosphatidylcholine, dioctanoyl phosphatidylcholine, dicaproyl phosphatidylcholine, distearoyl phosphatidylglycerol and sodium salt thereof, dipalmitoyl phosphatidylglycerol and sodium salt thereof, L-$\alpha$-dimyristoyl phosphatidylglycerol and sodium salt thereof, dilauroyl phosphatidylglycerol, dicaprinoyl phosphatidylglycerol, dioctanoyl phosphatidylglycerol, dicaproyl phosphatidylglycerol, distearoyl phosphatidylethanolamine, dipalmitoyl phosphatidylethanolamine, dioleoyl phosphatidylethanolamine, dimyristoyl phosphatidylethanolamine, dilauroyl phosphatidylethanolamine, bi-distearoyl phosphatidylglycerol and sodium salt thereof, bi-dipalmitoyl phosphatidylglycerol and sodium salt thereof, bi-dimyristoyl phosphatidylglycerol and sodium salt thereof, bi-dilauroyl phosphatidylglycerol, distearoyl phosphatidylinositol, dipalmitoyl phosphatidylinositol, dioleoyl phosphatidylinositol, dimyristoyl phosphatidylinositol, dilauroyl phosphatidylinositol, palmitoyl oleoyl phosphatidylcholine, palmitoyl linoleoyl phosphatidylcholine, stearyl linoleoyl phosphatidylcholine, stearyl oleoyl phosphatidylcholine, arachidonic stearoyl phosphatidylcholine, DSPE-PEG, DPPE-PEG, DMPE-PEG and DLPE-PEG, wherein the molecular weight of PEG in DSPE-PEG, DPPE-PEG, DMPE-PEG and DLPE-PEG is from 100 to 10000.

5. The drug delivery system according to any one of claims 1 to 4, wherein the said delivery system further comprises organic solvents selected from at least one of ethanol, methanol, propanol, propylene glycol, methyl-2-pyrrolidone, n-butanol, tert butyl alcohol, acetone, ethyl acetate and isopropyl ether.

6. The drug delivery system according to any one of claims 1 to 5, wherein the said delivery system further comprises oils, and the weight ratio between the oils and phospholipid is between 1:0.1 and 1:10, the said oils are selected from at least one of MCT, structured-oil, tocopherol, tocopheryl acetate, oleinic acid, ethyl oleate, soybean oil, safflower oil, olive oil, octanoic acid, decanoic acid, lauric acid, palmitinic acid, linoleic acid, linolenic acid, docosahexaenoic acid, deep-sea fish oil and plant volatile oil.

7. The drug delivery system according to any one of claims 1 to 6, wherein, the said drug delivery system further comprises ligand and antibody, the said ligand is selected from galactose derivates, transferrin derivates, folic acid and derivates thereof, glucosamine derivates and RGD and derivates thereof and so on, the said antibody is selected from murine, humanized, recombine murine or recombine humanized antibody.

8. The drug delivery system according to any one of claims 1 to 7, wherein, the said drug delivery system further comprises cholesterol and/or sitosterol.

9. The drug delivery system according to any one of claims 1 to 8, wherein, the said drug delivery system further comprises positively charged substances selected from chitosan, octadecylamine, fatty amine, cholesterol-based polyamine cationic lipids or derivatives thereof.

10. The drug delivery system according to any one of claims 1 to 9, wherein, the said drug delivery system further comprises pH regulator.

11. The drug delivery system according to any one of claims 1 to 8, wherein, the said pH regulator is selected from at least one of organic acid, organic base, inorganic acid or inorganic base, such as citric acid, lactic acid, fumaric acid, tartaric acid, acetic acid, glucanic acid, lactobionic acid, sorbic acid, succinic acid, maleic acid, ascorbic acid, oxalic acid, formic acid, benzenesulfonic acid, benzoic acid, aspartic acid, hydrochloric acid, sulphuric acid, phosphoric acid, nitric acid, sodium hydroxide, arginine, lysine.

12. A method for manufacturing the drug delivery system according to any one of claims 1 to 11, **characterized by** comprising the following steps:

    1) preparing the phospholipid-containing phase;
    2) preparing the protein-containing aqueous phase; and
    3) mixing the phospholipid-containing phase and the protein-containing aqueous phase, and extruding optionally at 0-60°C under a pressure of 400-40000psi, and then homogenizing.

13. The method according to claim 12, wherein the said protein-containing aqueous phase is 0.005-50% ( g/ml) protein aqueous solution.

14. The method according to claim 12 to 13, wherein the said homogenization is high press homogenization or microfluidic homogenization.

15. The method according to any one of claims 12 to 14, wherein the said method further comprises freeze drying or spray drying.

16. The method according to claim 15, wherein an additive used in the freeze drying or spray drying method is selected from at least one of sucrose, lactose, glucose, mannitol, dextran, trehalose, xylitol, maltose, fructose, protein, chitosan, glycine, citral and sodium chloride.

17. The method according to any one of claims 12 to 16, wherein the said method further comprises filtration sterilization.

18. Use of the protein-phospholipid dispersion in the manufacture of a drug delivery system.

**Figure 1**

PEG-THS

PEG-CHS

PEG-CHM

n=5~500 (Molecular weight of PEG is 300~30000)

**Figure 2**

**Figure 3**

**Figure 4**

Figure 5

Figure 6

**Figure 7**

**Figure 8**

**Figure 9**

**Figure 10**

Figure 11

# INTERNATIONAL SEARCH REPORT

| | International application No. |
|---|---|
| | PCT/CN2009/000063 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: A61K 47/-; A61K 9/-

Documentation searched other than minimum documentation to the extent that such documents are included in the fields searched

Electronic data base consulted during the international search (name of data base and, where practicable, search terms used)

WPI; EPODOC; PAJ; CNKI; CA; CNPAT;

protein; phospholipid; phosphatide; liposome; nano+; drug delivery; dispers+; homogeniz+

**C. DOCUMENTS CONSIDERED TO BE RELEVANT**

| Category* | Citation of document, with indication, where appropriate, of the relevant passages | Relevant to claim No. |
|---|---|---|
| X | WO 2004043363 A2 (AZAYA THERAPEUTICS, INC.), 27 May 2004(27.05.2004) Examples 1-16, claims 1-32 | 1-18 |
| X | WO 2006109936 A1 (REGERON, INC.), 19 October 2006(19.10.2006) Example I, Examples III-V, claims 1-21 | 1-18 |
| A | WO 2006033679 A2 (CHIMERACORE, INC.), 30 March 2006(30.03.2006) the whole document | 1-18 |
| A | CN 1511589 A (CHINESE ACAD SCI CHEM INST), 14 July 2004(14.07.2004) the whole document | 1-18 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 Apr.2009(07.04.2009) | **23 Apr. 2009 (23.04.2009)** |

| Name and mailing address of the ISA/CN | Authorized officer |
|---|---|
| The State Intellectual Property Office, the P.R.China 6 Xitucheng Rd., Jimen Bridge, Haidian District, Beijing, China 100088 Facsimile No. 86-10-62019451 | **WEI, Jun** Telephone No. (86-10)62413625 |

Form PCT/ISA/210 (second sheet) (April 2007)

## INTERNATIONAL SEARCH REPORT
Information on patent family members

| International application No. |
|---|
| PCT/CN2009/000063 |

| Patent Documents referred in the Report | Publication Date | Patent Family | Publication Date |
|---|---|---|---|
| WO 2004043363 A2 | 27.05.2004 | CA 2505520 A | 27.05.2004 |
| | | AU 2003287526 A1 | 03.06.2004 |
| | | US 2004247660 A1 | 09.12.2004 |
| | | EP 1585504 A2 | 19.10.2005 |
| | | JP 2006508126 T | 09.03.2006 |
| | | US 2007166368 A1 | 19.07.2007 |
| | | US 7179484 B2 | 20.02.2007 |
| WO 2006109936 A1 | 19.10.2006 | KR 20080000602 A | 02.01.2008 |
| | | JP 2008534580 T | 28.08.2008 |
| | | US 2008213346 A1 | 04.09.2008 |
| WO 2006033679 A2 | 30.03.2006 | CA 2567741 A | 30.03.2006 |
| | | AU 2005287383 A1 | 30.03.2006 |
| | | US 2006292174 A1 | 28.12.2006 |
| | | EP 1773303 A2 | 18.04.2007 |
| | | JP 2008500364 T | 10.01.2008 |
| CN 1511589 A | 14.07.2004 | NONE | |

Form PCT/ISA/210 (patent family annex) (April 2007)

# INTERNATIONAL SEARCH REPORT

International application No.

PCT/CN2009/000063

A.    CLASSIFICATION OF SUBJECT MATTER

A61K47/42 (2006.01) i

A61K47/24 (2006.01) i

A61K9/10 (2006.01) i

A61K9/19 (2006.01) i

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200380109606
- CN 97199720
- CN 20031023461X
- CN 02811017X
- CN 03108361
- CN 200610077006
- CN ZL01119258
- CN ZL03108361
- CN 200610037609

- CN 02129204
- CN 02160028
- CN ZL94191101
- CN ZL96190332
- US 2007082838 A1
- CN 200610121794
- CN 031083617A
- CN 200310123461X